# EUROPÄISCHE PATENTSCHRIFT

(11) **EP 2 726 121 B1**
(45) Veröffentlichungstag und Bekanntmachung des Hinweises auf die Patenterteilung: **11.03.2020**
(21) Anmeldenummer: 12730398.0
(22) Anmeldetag: 29.06.2012
(51) Int. Cl.: A61M 1/16, A61J 1/10

(54) **BEHÄLTER, VERWENDUNG, DIALYSEGERÄT ODER ZUBEREITUNGSEINHEIT SOWIE VERFAHREN ZUR HERSTELLUNG EINES KONZENTRATS**
CONTAINER, USE, DIALYSIS DEVICE OR PREPARATION UNIT AND METHOD FOR PRODUCING A CONCENTRATE
CONTENANT, UTILISATION, APPAREIL DE DIALYSE OU UNITÉ DE PRÉPARATION ET PROCÉDÉ POUR LA PRÉPARATION D'UN CONCENTRÉ

(30) Priorität: 01.07.2011 DE 102011106248; 01.07.2011 US 201161503808 P
(43) Veröffentlichungstag der Anmeldung: 07.05.2014
(60) Teilanmeldung: 20154634.8
(73) Patentinhaber: Fresenius Medical Care Deutschland GmbH, 61352 Bad Homburg (DE)
(72) Erfinder: WIESEN, Gerhard, 61352 Bad Homburg (DE); KLÖFFEL, Peter, 97720 Nüdlingen (DE); DUMONT D'AYOT, Francois, F-69005 Lyon (FR); LAFFAY, Philippe, F-69110 Sainte Foy Les Lyon (FR); LUAIRE, Benoît, F-69210 Sourcieux les Mines (FR)
(74) Vertreter: Herrmann, Uwe
(86) Internationale Anmeldenummer: PCT/EP2012/002738
(87) Internationale Veröffentlichungsnummer: WO 2013/004362

(56) Entgegenhaltungen:
- DE-B3- 10 313 965
- DE-T2- 60 207 683
- FR-A1- 2 766 797
- US-A1- 2011 120 302
- US-B1- 6 872 197

## Beschreibung

Die vorliegende Erfindung betrifft einen Behälter enthaltend wenigstens ein Trockenkonzentrat.

Im Rahmen der Durchführung einer Dialysebehandlung, insbesondere einer Hämodialysebehandlung wird üblicherweise ein basisches Konzentrat verwendet, das meist als Trockenkonzentrat vorliegt, das aus Bicarbonat besteht oder dieses enthält. Dieses Konzentrat befindet sich beispielsweise in einem Behälter, wie beispielsweise einem Beutel oder einer Kartusche, die mit dem Dialysegerät verbunden wird. Aus diesem Trockenkonzentrat wird sodann durch die Zufuhr hochreinen Wassers eine gesättigte Lösung bestehend aus oder enthaltend Bicarbonat hergestellt. Dieses basische Konzentrat kann aus dem Behälter abgeleitet werden und zur Herstellung der eigentlichen Dialyselösung herangezogen werden, wobei mit der Ableitung des Konzentrates begonnen werden kann, wenn sich noch festes Trockenkonzentrat in dem Behälter befindet.

Bei der Herstellung einer Dialyselösung wird üblicherweise außer dem genannten basischen Konzentrat ein sogenanntes saures Konzentrat verwendet. Dieses besteht aus einer Lösung mehrerer Komponenten, die meist in unterschiedlichen Mengen und/oder Konzentrationen vorliegen. Typische Bestandteile sind NaCl als Hauptbestandteil und in geringeren Mengen die übrigen Elektrolyte, wie CaCl₂ und MgCl₂. Die Spezifikationen für die Konzentrationen der Elektrolyte sind sehr eng, was zur Folge hat, dass vor der Verwendung des sauren Konzentrats in der Portionierungs- bzw. Dosiereinheit zur Herstellung der Dialyselösung alle Komponenten des sauren Konzentrats vollständig gelöst sein müssen. Dies kann nicht wie beim basischen Konzentrat im Durchfluss erfolgen. Vielmehr müssen hier spezielle Mischvorrichtungen, wie z. B. Rührer verwendet werden, um diese vollständige Auflösung in einem annehmbaren Zeitraum zu gewährleisten.

Das genannte saure Konzentrat wird vor diesem Hintergrund üblicherweise als Flüssigkonzentrat in einem Kanister geliefert, aus dem das Flüssigkonzentrat mittels des Dialysegerätes oder einer Zubereitungseinheit abgezogen und zur Herstellung der fertigen Dialyselösung verwendet wird. Ein Nachteil bei der Verwendung derartiger Kanister besteht in dem vergleichsweise umständlichen Handling sowie in dem relativ hohen Gewicht, was Nachteile hinsichtlich der Transport- und Lagerkosten mit sich bringt.

In Kliniken, in denen eine größere Anzahl von Behandlungsplätzen vorliegt, kommen mitunter auch Ringleitungen zum Einsatz, die mit einer zentralen Versorgungseinheit in Verbindung stehen. In dieser zentralen Versorgungseinheit wird das saure Flüssigkonzentrat unter Zuhilfenahme spezieller Mischvorrichtungen hergestellt oder bereitgestellt und dann in die Ringleitung eingespeist. An den Dialysegeräten bzw. an den Behandlungsplätzen wird es aus der Ringleitung abgezogen und steht dann an dem Dialysegerät zur Zubereitung der gebrauchsfertigen Dialyselösung bereit.

Ein Nachteil dieser Vorgehensweise besteht darin, dass die Desinfektion und Reinigung eines solchen System aufwändig, teuer und umweltbelastend ist und zudem darin, dass sich diese Art der Konzentratversorgung aufgrund der relativ hohen Kosten nur für größere Behandlungszentren lohnt.

Aus der Druckschrift US2011/0120302A1 ist ein Verfahren zum Entfernen von Gas aus einem Behälter mit Pulverkonzentrat, welcher bei der Hämodialyse zum Einsatz kommt, bekannt. Zudem offenbart die Druckschrift US6872197B1 einen Behälter zur Verabreichung von Flüssigkeiten. Darüber hinaus offenbart die DE60207683T2 einen Behälter, welcher dazu ausgelegt ist, mit einer Fluidaufbereitungsvorrichtung verbunden zu werden. Die Druckschrift FR2766797A1 offenbart einen Behälter für Trockenkonzentrat. Weiterhin ist aus der DE10313965B3 eine Vorrichtung zur Herstellung von Dialysekonzentraten, insbesondere sauren Konzentraten, bekannt.

Der vorliegenden Erfindung liegt die Aufgabe zugrunde, eine Möglichkeit der Bereitstellung eines sauren Konzentrats zu schaffen, die mit geringen Kosten verbunden ist und einfach durchführbar ist.

Diese Aufgabe wird durch ein Dialysegerät bzw. ein Verfahren mit den Merkmalen der unabhängigen Ansprüchegelöst.
Gemäß einem Aspekt der Erfindung ist vorgesehen, dass das in dem Behälter befindliche Trockenkonzentrat derart ausgeführt ist, dass es bei seiner Lösung in einer Flüssigkeit, vorzugsweise in Wasser ein saures Flüssigkonzentrat oder einen Teil eines sauren Flüssigkonzentrats bildet, das zur Herstellung einer Dialyselösung geeignet ist.

Der vorliegenden Erfindung liegt somit der Gedanke zugrunde, das saure Flüssigkonzentrat durch Auflösen eines in einem Behälter, wie z. B. in einem Beutel oder in einer Kartusche befindlichen sauren Trockenkonzentrats bereitzustellen. Dadurch kann der Transport von schweren Kanistern zum Dialysegerät sowie die Schaffung aufwändiger Ringleitungssysteme vermieden werden. Ausreichend ist es, den Behälter gemäß der vorliegenden Erfindung mit dem Dialysegerät oder mit einer vorzugsweise lokalen Zubereitungseinheit, die sich vorzugsweise in räumlicher Nähe zu dem Dialysegerät befindet, fest zu verbinden und dann vor Ort, d.h. am Behandlungsplatz das saure Flüssigkonzentrat durch Auflösen des genannten Trockenkonzentrats herzustellen.

Die vorliegende Erfindung bezieht sich somit auf die Herstellung des üblicherweise im Rahmen der Dialysebehandlung und zur Herstellung der gebrauchsfertigen Dialyselösung eingesetzten Säurekonzentrats. Dieses saure Flüssigkonzentrat enthält vorzugsweise die Ionen Na⁺, Ca²⁺, Mg²⁺, K⁺, Cl⁻ und ggf. weitere Ionen und Zusätze, wie eine oder mehrere Säuren, Puffer, Acetat, Glucose etc.

Wird eine Zubereitungseinheit verwendet, befindet sich diese vorzugweise nicht an einem zentralen Ort, sondern ist lokal angeordnet, beispielsweise an oder in der

Nähe des Dialysegerätes bzw. Behandlungsplatzes. Denkbar ist es, dass je Dialysegerät eine derartige Zubereitungseinheit vorgesehen ist.

Erfindungsgemäß ist es somit möglich, ähnlich wie auch das basische Konzentrat das saure Trockenkonzentrat in einem mit dem Dialysegerät oder einer Zubereitungseinheit vorzugsweise fest verbindbaren Disposable oder sonstigen Behältnis vorzulegen. Denkbar ist es, dass das Behältnis ein oder mehrere Verbindungselemente aufweist, die mit einem oder mehreren Konnektionsmitteln des Dialysegerätes oder der Zubereitungseinheit durch eine Steckverbindung oder anderweitig und unabhängig von der Art der Verbindung vorzugsweise lösbar verbindbar sind. Die Verbindung zwischen dem Behältnis und dem Dialysegerät oder der Zubereitungseinheit ist vorzugsweise fluiddicht und lösbar ausgebildet, so dass das Behältnis nach seinem Gebrauch wieder entfernt und durch ein neues, mit Trockenkonzentrat gefülltem Behältnis ersetzt werden kann.

Das Trockenkonzentrat kann dann beispielsweise durch in dem Dialysegerät oder in einer Zubereitungseinheit bereits vorhandene Vorrichtungen, wie Pumpen und Druckluftkompressoren zur Auflösung gebracht werden und dann direkt zur Herstellung der gebrauchsfertigen Dialyselösung verwendet werden.

Alternativ oder zusätzlich dazu können auch vorzugsweise kleinere lokale Mischeinheiten, die im Rahmen der vorliegenden Erfindung auch als Zubereitungseinheiten bezeichnet werden, verwendet werden, an die das Konzentratbehältnis, d.h. der erfindungsgemäße Behälter angeschlossen werden kann. Darin erfolgt die Auflösung des sauren Trockenkonzentrats, wodurch das saure Flüssigkonzentrat erzeugt wird, das dann in geeigneter Weise dem Dialysegerät zugeführt bzw. zur Herstellung der fertigen Dialyselösung verwendet werden kann.

Der erfindungsgemäße Behälter ist derart ausgebildet, dass er Verbindungsmittel aufweist, mittels derer der Behälter an ein Dialysegerät oder an eine Zubereitungseinheit konnektierbar ist. Diese Verbindungsmittel können derart ausgeführt sein, dass sie mit Konnektionsmitteln des Dialysegerätes oder der Zubereitungseinheit eine zuverlässige Anbringung des Behälters an dem Dialysegerät bzw. an der Zubereitungseinheit gewährleisten. Die Konnektion kann derart ausgebildet sein, dass sie flüssigkeitsdicht und/oder gasdicht ausgebildet ist.

Die Verbindungsmittel und die Konnektionsmittel können derart ausgebildet sein, dass der Behälter durch eine Steckverbindung an dem Dialysegerät bzw. an der Zubereitungseinheit angeordnet werden kann. Auch ist es möglich, die Verbindungsmittel am Behälter sowie die Konnektionsmittel am Dialysegerät oder an der Zubereitungseinheit derart auszubilden, dass die Konnektion durch eine Dreh- bzw. durch eine Schwenkbewegung des Behälters, ggf. vergleichbar mit einem Bajonettverschluß erzielt wird.

Die Verbindungsmittel des erfindungsgemäßen Behälters können beispielsweise derart mit Konnektionsmitteln des Dialysegerätes oder einer sonstigen Zubereitungseinheit zusammenwirken bzw. kommunizieren, dass zum einen ein Verbindungsbereich zwischen Konnektionsmitteln und Verbindungsmitteln hergestellt wird, durch die das zur Auflösung des Trockenkonzentrats verwendete Medium, insbesondere Flüssigkeit, vorzugsweise Wasser sowie zusätzlich Gas, vorzugsweise Luft eingeleitet wird. Diese eingeleitete Flüssigkeit bzw. das eingeleitete Wasser sowie die eingeleitete Luft bzw. Gas kann dann ausgehend von dem Verbindungsmittel des Behälters durch einen Schlauch, durch einen Kanal, durch ein Rohr etc. vorzugsweise in einen unteren Bereich des Behälters eingeleitet werden. Durch diese Konnektionsstelle kann vorzugsweise auch das fertige Flüssigkonzentrat aus dem Behältnis abgezogen werden.

Die Verbindungsmittel und die Konnektionsmittel können des Weiteren einen zweiten Verbindungsbereich bilden, durch den ein Fluid, insbesondere ein Gas und besonders bevorzugt Luft aus dem Behälter abgezogen wird.

In einer bevorzugten Ausgestaltung der Erfindung wird zum Zwecke des Lösens des Trockenkonzentrats nicht nur Wasser, sondern auch Luft durch die Verbindungsmittel des Behältnisses und durch die Konnektionsmittel des Dialysegerätes bzw. der Zubereitungseinheit bzw. durch eine entsprechende Fluidverbindung dieser Mittel und weiter vorzugsweise durch einen Schlauch oder dergleichen in den Behälter eingeleitet.

Damit diese Luft entweichen kann, weisen die Verbindungsmittel sowie die Konnektionsmittel einen zweiten Verbindungsbereich auf, der eine zweite Fluidverbindung zwischen Behältnis und Dialysegerät bzw. Zubereitungseinheit bildet und durch den die Luft aus dem Behälter z. B. in die Atmosphäre oder in das Dialysegerät bzw. in die Zubereitungseinheit und von diesem z.B. in die Atmosphäre abgeleitet werden kann.

Eine exemplarische Ausgestaltung für die Verbindungsmittel des Behälters einerseits und für Konnektionsmittel des Dialysegerätes bzw. einer Zubereitungseinheit andererseits findet sich in der EP 1 344 550 B1 auf die hiermit vollumfänglich Bezug genommen wird und deren Offenbarungsgehalt hiermit zum Gegenstand der vorliegenden Erfindung gemacht wird. Wie dies aus dieser Druckschrift hervorgeht, weisen die Verbindungsmittel des Behälters jeweils Verbinderteile auf, die jeweils mit einem korrespondierenden Verbinderteil des Konnektionsmittels konnektierbar sind. Diese Verbinderteile weisen jeweils einen Durchlass auf, durch den gemäß der vorliegenden Erfindung Luft und das Lösungsmedium zum Lösen des Trockenkonzentrats, insbesondere Wasser hindurchströmt und dann in den Behälter gelangt.

Das andere Verbinderteil des Verbindungsmittels weist ebenfalls eine Öffnung bzw. einen Durchlass auf, der dazu dient, Luft aus dem Behälter während des Lösevorgangs abzuleiten.

Abweichend von der Lehre der EP 1 344 550 B1 ist vorzugsweise im Rahmen der vorliegenden Erfindung vorgesehen, dass ein Verbinderteil zur Zufuhr von Luft und des Lösungsmittels, insbesondere Wasser dient sowie auch zur Abfuhr des flüssigen Konzentrats dient. Das andere Verbinderteil dient im Rahmen der vorliegenden Erfindung vorzugsweise nur zur Abfuhr von Gas bzw. Luft aus dem Behälter.

Das genannte, in dem Behälter befindliche Trockenkonzentrat kann beispielsweise NaCl und/oder CaCl₂ und/oder MgCl₂ und/oder KCl und/oder weitere Elektrolytsalze und/oder Zitronensäure und/oder eine oder mehrere weitere Säuren und/oder Glucose und/oder Acetat oder andere Ionen enthalten bzw. aus einem oder mehreren dieser Stoffe bestehen.

Als weitere Ausführungsform bzw. Aspekt der Erfindung ist möglich, dass der Behälter ein solches Volumen aufweist, dass außer dem Trockenkonzentrat Flüssigkeit, vorzugsweise Wasser in einem Volumen von 2 bis 15 Litern oder von 4 bis 15 Litern, vorzugsweise in einem Volumen von 4 bis 15 Litern aufnehmbar bzw. aufgenommen ist, um das saure Flüssigkonzentrat durch Auflösen des Trockenkonzentrats herzustellen, und/oder dass der Behälter ein Gesamtvolumen im Bereich von 4 bis 15 Litern aufweist.

Denkbar ist es insbesondere, dass das Trockenkonzentrat in einer Menge in dem Behälter enthalten ist, das sich bei dessen Auflösung in einem Volumen von 4 bis 15 Litern Flüssigkeit, vorzugsweise Wasser, ein flüssiges Konzentrat ergibt, wobei das Trockenkonzentrat vorzugsweise vollständig gelöst ist. Dieses wird zur Herstellung der fertigen Dialyselösung verdünnt, so dass die Stoffe bezogen auf das Volumen der fertigen Dialyselösung vorzugsweise in den folgenden Konzentrationsbereichen vorliegen:

| | |
|---|---|
| NaCl: | 110 - 170 mmol/l, vorzugsweise 130- 150 mmol/l |
| KCl: | 0,7 - 4,3 mmol/l, vorzugsweise 1,0 - 4,0 mmol/l |
| CaCl₂: | 0,7 - 2,0 mmol/l, vorzugsweise 1,0 - 1,75 mmol/l |
| MgCl₂: | 0,3 - 1,2 mmol/l, vorzugsweise 0,5 - 1,0 mmol/l |
| Glucose: | 0,8 - 2,2 g/l, vorzugsweise 1,0 - 2,0 g/l |
| Zitronensäure: | 0,1 - 20 mmol/l, vorzugsweise 1,0 - 15,0 mmol/l, |

Alle Werte beziehen sich auf die fertige Dialyselösung. Denkbar ist es beispielsweise, dass 34 Liter einer Mischung aus Wasser und dem basischen Konzentrat mit 1

Liter des sauren Flüssigkonzentrats gemischt werden, um 35 Liter der gebrauchsfertigen Dialyselösung zu erhalten. Dieses Mischungsverhältnis gilt selbstverständlich nicht nur für das oben genannte Beispiel, sondern kann grundsätzlich als geeignetes Mischungsverhältnis angenommen werden.

Die bevorzugte Volumenkapazität des erfindungsgemäßen Behälters liegt vorzugsweise im Bereich von 4 bis 15 Litern.

Das Trockenkonzentrat und somit auch das daraus gebildete saure Konzentrat kann einen, mehrere oder alle der vorgenannten Stoffe enthalten. Auch ist es möglich, dass das Trockenkonzentrat und somit auch das daraus gebildete saure Konzentrat weitere als die genannten Stoffe enthält.

Vorzugsweise ist vorgesehen, dass das saure Flüssigkonzentrat einen pH-Wert < 7,0 aufweist und/oder dass das Trockenkonzentrat wenigstens eine Säure enthält.

Denkbar ist es, dass sich außer den explizit genannten Substanzen auch noch weitere Stoffe in dem Trockenkonzentrat befinden, wie beispielsweise weitere Elektrolyte, Puffer, Säuren etc.

Der Behälter kann starre und/oder flexible Wandungen aufweisen. Er ist vorzugsweise als Beutel ausgeführt oder auch als Kartusche, die feste Wandungen aufweist.

Auch eine Kombination aus festen und flexiblen Wandungen ist denkbar. So kann der Behälter beispielsweise in dem oder den Bereichen, in denen Flüssigkeit und/oder Gas zugeführt und/oder das saure Flüssigkonzentrat abgeführt wird, eine feste Wandung und im übrigen eine flexible Wandung aufweisen.

Weiterhin ist es denkbar, dass es sich bei dem Behälter um ein Disposable, d.h. um einen Wegwerfartikel handelt. Darunter ist zu verstehen, dass der Behälter nach Ablauf seiner Nutzungsdauer, d.h. beispielsweise nach ca. 1 bis 3 Einsätzen bzw. Behandlungen verworfen, d.h. nicht wieder verwendet wird. Denkbar ist es, die Behälter in Form von Beuteln oder Kartuschen oder dergleichen anzuliefern, darin das saure Flüssigkonzentrat herzustellen und dieses für eine oder mehrere Dialysebehandlungen zu verwenden. Ist es verbraucht, wird das Behältnis weggeworfen.

In dem Behälter kann das Trockenkonzentrat in einer Menge von 0,5 kg bis 6 kg, vorzugsweise in einer Menge von 0,75 kg bis 5,5 kg, weiter vorzugsweise in einer Menge von 1,0 kg bis 5,0 kg und besonders bevorzugt in einer Menge von 1,3 bis 4,2 kg vorliegen. Derartige Behälter erlauben ein problemloses Handling, sind leicht transportierbar und lagerbar und eignen sich für die Durchführung einer oder mehrerer Hämodialysebehandlungen.

Es ist möglich, dass das Trockenkonzentrat derart zusammengesetzt ist, dass das bei dessen Lösung in einem Volumen von 2 bis 15 Litern oder von 4 bis 15 Litern, vorzugsweise in einem Volumen von 4 bis 15 Litern Flüssigkeit, vorzugsweise Wasser, erhaltene saure Flüssigkonzentrat einen pH-Wert < 7,0 aufweist.

Denkbar ist es, dass in dem Behälter vor dem Lösen des Trockenkonzentrats keine flüssigen Stoffe enthalten sind. Von der Erfindung ist jedoch auch der Fall umfasst, dass in dem Trockenkonzentrat wenigstens auch eine flüssige Komponente vorhanden ist, wie beispielsweise eine Säure. Der Begriff "Trockenkonzentrat" ist somit dahingehend zu verstehen, dass sich das Trockenkonzentrat nicht im vollständig gelösten Zustand befindet, d.h. zumindest teilweise, vorzugsweise vollständig aus einem oder mehreren Feststoffen besteht.

Weiterhin kann der Behälter derart ausgebildet sein, dass ein Volumen des sauren Flüssigkonzentrats im Bereich von 1 bis 15 Litern, vorzugsweise im Bereich von 2 bis 14 Litern, weiter vorzugsweise im Bereich von 3 bis 13 Litern und besonders bevorzugt im Bereich von 4 bis 15 Litern in dem Behälter aufnehmbar oder aufgenommen ist.

In weiterer Ausgestaltung der Erfindung weist der Behälter wenigstens ein Einführmittel auf, durch das zum Zwecke des Lösens des in dem Behälter befindlichen Trockenkonzentrats die Flüssigkeit, vorzugsweise Wasser, oder die Flüssigkeit, vorzugsweise Wasser, und ein Gas, vorzugsweise Luft in den Behälter einleitbar ist. Bei dem Einführmittel kann es sich beispielsweise um einen Schlauch, ein Kanal, ein Rohr oder um eine sonstige Leitung handeln.

Grundsätzlich können die Einführmittel derart ausgebildet sein, dass die Flüssigkeit oder die Flüssigkeit und das Gas in einen Bereich des Behälters eingeführt wird, in dem sich das Trockenkonzentrat befindet, wie beispielsweise in einem in Betriebsposition unteren Bereich des Behälterinnenraums.

Die Zufuhr der Flüssigkeit und/oder des Gases in den Behälter muss also derart ausgebildet sein, dass das Ende des Einführmittels in dem Trockenkonzentrat endet, was den Vorteil mit sich bringt, dass die Zufuhr von Flüssigkeit oder von Flüssigkeit und Gas ggf. zu einer Aufwirbelung des Trockenkonzentrats führt, wodurch sich dessen Auflösung beschleunigt.

Bei dem zugeführten Gas handelt es sich im Rahmen der vorliegenden Erfindung vorzugsweise um sterilfiltrierte Luft.

Die oder das Einführmittel sind vorteilhafterweise somit derart dimensioniert, dass sie bis in das Trockenkonzentrat hinein ragen. So ist es möglich, dass ein Schlauch oder eine sonstige Leitung zum Beispiel von einer Wandung bzw. einem Anschlussbereich des Behälters in das Behälterinnere hineinragt.

In weiterer Ausgestaltung der Erfindung ist vorgesehen, dass der Behälter in wenigstens einem Bereich Wandbereiche aufweist, zwischen denen sich ein talförmiger oder muldenförmiger Bereich ausbildet, wobei das Trockenkonzentrat in der Betriebsposition des Behälters zumindest auch in dem talförmigen Bereich vorliegt. Auf diese Weise kann sichergestellt werden, dass das Trockenkonzentrat in den genannten talförmigen Bereich "nachrutscht" und damit an einer zentralen Position vorliegt, in der günstige Bedingungen für das Auflösen des Trockenkonzentrats vorherrschen.

Vorzugsweise ist somit vorgesehen, dass das Behältnis zumindest eine oder mehrere trichterförmige Verengungen aufweist, und zwar im unteren Endbereich des Behältnisses. Damit wird gewährleistet, dass sich das nicht gelöste Trockenkonzentrat während des gesamten Lösungsvorgangs direkt an der Fluid- bzw. Flüssigkeits- und Luftzugabestelle befindet und so ständig aufgewirbelt wird. Insbesondere durch diese Maßnahme ist die Dauer zur vollständigen Auflösung des Konzentrats auf einen günstigen Zeitraum reduziert.

Vorzugsweise erstreckt sich das Einführmittel, d.h. der Schlauch, die Leitung oder dergleichen bis in den talförmigen Bereich bzw. bis in die trichterförmige Verengung.

Insbesondere kann vorgesehen sein, dass sich das Einführmittel bis in den talförmigen Bereich erstreckt und vorzugsweise in diesem Bereich wenigstens eine Öffnung, vorzugsweise wenigstens eine Auslass- und/oder wenigstens eine Einlassöffnung aufweist, wobei vorzugsweise vorgesehen ist, dass durch die Auslassöffnung im Betrieb eine Flüssigkeit, vorzugsweise Wasser sowie Gas, vorzugsweise Luft in den Behälter eingeführt wird und dass durch die Einlassöffnung im Betrieb das saure Flüssigkonzentrat aus dem Behälter abgeführt wird und/oder dass die Auslassöffnung und die Einlassöffnung durch ein und dieselbe Öffnung gebildet werden.

Weiterhin kann vorgesehen sein, dass das Einführmittel ein Verschlusselement aufweist, das derart angeordnet und ausgeführt ist, dass das Eindringen des Trockenkonzentrates in das Einführmittel verhindert wird. Auf diese Weise ist sichergestellt, dass in die Einführmittel kein Feststoff, sondern nur Gas bzw. Luft und/oder Flüssigkeit bzw. Wasser oder das saure Flüssigkonzentrat gelangen kann.

Das spezifische Gewicht des Verschlusselementes des Einführmittels liegt vorzugsweise im Bereich < 11 kN/m³, vorzugsweise im Bereich < 10 kN/m³ und besonders bevorzugt im Bereich von < 9,7 kN/m³.

In weiterer Ausgestaltung der Erfindung weist der Behälter wenigstens ein Auslassmittel auf, mittels dessen das saure Flüssigkonzentrat aus dem Behälter abführbar ist. Bei diesem Auslassmittel kann es sich beispielsweise um einen Schlauch oder um eine sonstige Leitung handeln, die vorzugsweise in das Innere des Behälters hineinragt. Auch ist es möglich, dass Auslassmittel einfach als Öffnung im Behälter auszubilden.

Insbesondere kann vorgesehen sein, dass das Auslassmittel als zu dem Einführmittel separates Bauteil ausgebildet ist und/oder dass das Einführmittel und das Auslassmittel an unterschiedlichen Positionen oder Seiten des Behälters, vorzugsweise an gegenüberliegenden Seiten des Behälters oder an derselben Seite des Behälters angeordnet sind.

Denkbar ist es, dass das Auslassmittel teilweise oder vollständig durch das Einführmittel gemäß den vorstehend beschriebenen Möglichkeiten gebildet wird. Bei dem Einführmittel sowie bei dem Auslassmittel kann es sich somit um ein und dasselbe Element des Behälters handeln. In Betracht kommt beispielsweise ein Schlauch oder eine sonstige Leitung. Durch diese kann zum Zwecke des Auflösens des Trockenkonzentrats zunächst beispielsweise hochreines Wasser bzw. RO-Wasser und/oder vorzugsweise gereinigte bzw. filtrierte Luft gefördert werden. Ist das Trockenkonzentrat vorzugsweise vollständig aufgelöst, kann vorgesehen sein, dass das so gebildete saure Flüssigkonzentrat durch denselben Schlauch oder dieselbe Leitung abgeführt wird.

Das saure Konzentrat wird dem Dialysegerät zugeführt bzw. in dem Dialysegerät zur Herstellung einer fertigen Dialyselösung verwendet, indem das saure Konzentrat sowie vorzugsweise ein basisches Konzentrat mit Wasser zu der fertigen Dialyselösung gemischt wird. Dazu können eine oder mehrere Dosiereinrichtungen, wie beispielsweise Pumpen oder dergleichen verwendet werden, die das Konzentrat bzw. die Konzentrate in der geeigneten Menge dem Wasser bzw. dem beispielsweise durch eine Hauptleitung strömenden Wasserstrom zudosieren, so dass die fertige Dialyselösung die gewünschten Komponenten in der gewünschten Konzentration umfasst.

Denkbar ist es, dass dem RO-Wasser zunächst ein basisches Flüssigkonzentrat und sodann das erfindungsgemäße saure Flüssigkonzentrat beigemischt wird. Dabei kann vorgesehen sein, dass zu ca. 33 Teilen RO-Wasser jeweils in etwa ein Teil des basischen Flüssigkonzentrates und in etwa ein Teil des sauren Flüssigkonzentrats zudosiert werden.

Dabei kann vorgesehen sein, dass das RO-Wasser durch eine Hauptleitung strömt, die mit zwei Nebenleitungen kommuniziert, von denen in einer das basische Flüssigkonzentrat und in der anderen das saure Flüssigkonzentrat vorliegt bzw. die mit den jeweiligen Behältern in Verbindung stehen, in denen die Konzentrate jeweils hergestellt werden.

Weiterhin kann vorgesehen sein, dass der Behälter wenigstens ein Entlüftungsmittel aufweist, das derart angeordnet und ausgeführt ist, dass Luft oder ein sonstiges Gas aus dem Behälter entweichen kann, was vorzugsweise dann der Fall ist, wenn die Flüssigkeit und/oder das Gas zum Zwecke des Auflösens des Trockenkonzentrats in den Behälter eingeleitet wird.

Das Entlüftungsmittel kann beispielsweise durch wenigstens eine Leitung und/oder durch wenigstens eine Membran bzw. einen Filter gebildet werden, die/der beispielsweise in der Behälterwandung oder in einer Leitung oder dergleichen angeordnet ist, die mit dem Behälterinnenraum in Verbindung steht, so dass Luft entweichen kann. Vorzugsweise handelt es sich bei dem Entlüftungsmittel um ein anderes Bauteil als bei dem Mittel zur Einfuhr von Wasser und Luft sowie zum Abführen des Konzentrats.

Weiterhin kann vorgesehen sein, dass der Behälter zumindest eine Codierung aufweist, mittels derer der Behälter und/oder das Trockenkonzentrat und/oder das flüssige saure Konzentrat identifizierbar ist. So ist es beispielsweise möglich, dass eine Erkennungseinheit beispielsweise an dem Dialysegerät oder an der Zubereitungseinheit oder auch in einem sonstigen beispielsweise handgehaltenen Gerät vorgesehen ist, mittels dessen die Codierung auslesbar ist. Auf der Grundlage dieser Information kann dann beispielsweise automatisiert oder manuell ein bestimmter Lösevorgang zum Auflösen des Trockenkonzentrats initiiert werden. Findet eine automatisierte Erkennung der Codierung statt, kann vorgesehen sein, dass ohne weitere Interaktion durch den Nutzer ein automatischer Lösungsvorgang zum Auflösen des Trockenkonzentrats eingeleitet wird. Ist dieser abgeschlossen, kann weiter vorgesehen sein, dass ggf. ebenfalls automatisiert eine Zudosierung des sauren Konzentrats zur Herstellung der fertigen Dialyselösung erfolgt.

Denkbar ist es, dass das Trockenkonzentrat in der Form eines Pulvers und/oder in der Form eines Granulates vorliegt. Wie bereits oben ausgeführt, kann das Trockenkonzentrat nur feste Stoffe enthalten. Von dem Begriff "Trockenkonzentrat" im Sinne der vorliegenden Erfindung ist jedoch auch der Fall umfasst, dass flüssige Komponenten in dem Behälter vorliegen.

Die Verbindungsmittel zur Konnektion des Behälters können derart ausgebildet sein, dass eine Verbindung des Behälters nur mit einem Konnektionsmittel eines Dialysegerätes oder einer Zubereitungseinheit bzw. eines bestimmten Typs eines Dialysegerätes bzw. einer Zubereitungseinheit für ein Dialysegerät möglich ist. So ist es denkbar, dass die Verbindungsmittel des Behälters und die Konnektionsmittel des Dialysegerätes oder der Zubereitungseinheit im Sinne eines Schlüssel-Schloss-Prinzips zusammenpassen bzw. speziell aneinander angepasst sind, so dass eine fehlerhafte Konnektion an nicht geeignete oder zur Verwendung des Behälters nicht vorgesehene Dialysegeräte oder Zubereitungseinheiten ausgeschlossen ist.

Bei dem Behälter kann es sich beispielsweise um einen sogenannten Standbodenbehälter handeln. Ein solcher Behälter ist üblicherweise durch wenigstens zwei Wandungen gekennzeichnet, die durch einen Boden miteinander verbunden sind, der vorzugsweise derart ausgebildet ist, dass der Behälter insgesamt stehen kann. Dabei ist es denkbar, dass dieser Standbodenbehälter im Rahmen der vorliegenden Erfindung derart verwendet wird, dass der Boden oben angeordnet ist, so dass sich beispielsweise ein auf dem Kopf stehendes Dreieck ergibt. Dieses kann, muss jedoch nicht genau vertikal stehend angeordnet sein, auch eine schräge Anordnung ist von der Erfindung mitumfasst. Vorzugsweise ist jedoch vorgesehen, dass die dem genannten Standboden gegenüberliegende Ecke des Behälters unten bzw. in einem unteren Bereich angeordnet ist, wenn der Behälter verwendet wird.

Ein solcher Standbodenbehälter weist den Vorteil auf, dass er im leeren Zustand flach ist, da der Standboden vorzugsweise derart ausgeführt ist, dass er eingefaltet werden kann. Im gefüllten Zustand stellt ein solcher Standbodenbehälter ein vergleichsweise großes Volumen bereit. Der Behälter ist vorzugsweise in der Art eines Standbodenbehälters ausgebildet und umfasst Ausführungen, bei denen der Standboden eine hinreichende Stabilität aufweist, so dass der Behälter theoretisch stehen kann, als auch solche, bei denen dies nicht der Fall ist.

Vorzugsweise ist der Standbodenbehälter derart ausgebildet, dass der Standboden im eingefalteten Zustand einen eingefalteten "Bodenabschnitt" aufweist, dessen Schenkel jedoch ungleich lang sind, so dass sich ein unsymmetrisches Dreieck ergibt, wenn der Behälter gefüllt bzw. entfaltet ist. Eine solche Ausführungsform führt dazu, dass beim Füllen des Behälters der oben befindliche "Standboden" aufgefaltet wird, wobei sich aufgrund der unsymmetrischen Ausbildung des gefalteten Standbodens eine nur geringe Verschiebung der Schwerkraft ergibt, so dass der V-förmige untere Bereich des Behälters auch in dieser unteren Position verbleibt, wenn der Behälter gefüllt ist.

Dadurch lässt sich eine effiziente Lösung des Trockenkonzentrates erreichen.

in einer denkbaren Ausgestaltung der Erfindung ist vorgesehen, dass der Behälter aus zwei Folien besteht, die miteinander in geeigneter Weise dichtend verbunden werden. So ist es beispielsweise denkbar, die Folien miteinander zu verschweißen, so dass das Flüssigkonzentrat in dem Behälter aufgenommen werden kann, das durch Lösen des Trockenkonzentrats entsteht.

Möglich ist es, eine Wandung bzw. Folie im Wesentlichen gerade auszuführen und die andere mit einem gefalteten Eckbereich bzw. Randbereich auszuführen, was den Vorteil mit sich bringt, dass der Behälter im eingefalteten Zustand sehr wenig Platz beansprucht und im ausgefalteten Zustand ein großes Volumen aufnehmen kann.

In einer bevorzugten Ausgestaltung der Erfindung besteht der Behälter somit genau aus zwei Folien, die sämtliche Wandungen des Behälters bilden.

Die Verwendung des Behälters ist vorzugsweise derart vorgesehen, dass der "Boden", der durch den Falz einer der Folien gebildet werden kann, bei Verwendung des Behälters oben bzw. in einem oberen Bereich angeordnet ist, so dass die diesem gegenüberliegende Seite des im Längsschnitt vorzugsweise dreieckförmigen Behälters unten angeordnet ist.

Die vorliegende Erfindung betrifft des Weiteren die Verwendung eines Behälters gemäß der vorliegenden Erfindung zur Herstellung eines sauren Flüssigkonzentrats, das seinerseits zur Herstellung einer Dialyselösung, vorzugsweise einer Dialyselösung für die Hämodialyse dient. Denkbar ist es, dass die Dialyselösung durch Zudosierung des sauren Flüssigkonzentrats und ggf. eines basischen Konzentrats zu einem Flüssigkeitsstrom oder zu einem Flüssigkeitsvolumen, vorzugsweise bestehend aus hochreinem Wasser bzw. RO-Wasser hergestellt wird. Diese Dialyselösung kann dann ggf. nach Zugabe weiterer Stoffe und ggf. einer geeigneten Temperierung als fertige Dialyselösung zur Durchführung einer Dialysebehandlung, vorzugsweise einer Hämodialysebehandlung verwendet werden.

Die Erfindung betrifft ferner die Verwendung eines in einem erfindungsgemäßen Behälter befindlichen sauren Flüssigkonzentrats zur Herstellung einer Dialyselösung, vorzugsweise einer Dialyselösung, die für die Hämodialyse verwendet wird.

Die vorliegende Erfindung betrifft des Weiteren ein Dialysegerät oder eine Zubereitungseinheit, wobei die Zubereitungseinheit zur Herstellung eines Konzentrats für eine Dialyselösung dient. Erfindungsgemäß ist vorgesehen, dass das Dialysegerät oder die Zubereitungseinheit mit einem erfindungsgemäßen Behälter konnektiert ist oder zur Konnektion mit einem derartigen Behälter geeignet ist.

Das Dialysegerät bzw. die Zubereitungseinheit ist somit geeignet, mit einem erfindungsgemäßen Behälter über geeignete Konnektionsmittel konnektiert zu werden. Vorzugsweise ist vorgesehen, dass das Dialysegerät bzw. die Zubereitungseinheit Mittel aufweist, mittels derer das in dem konnektierten Behälter befindliche Trockenkonzentrat vollständig aufgelöst werden kann, um auf diese Weise ein saures Flüssigkonzentrat bereitstellen zu können. Diese Mittel können derart ausgeführt sein, dass in den Behälter wenigstens eine Flüssigkeit, vorzugsweise hochreines Wasser bzw. RO-Wasser, oder wenigstens eine Flüssigkeit, vorzugsweise hochreines Wasser bzw. RO-Wasser, und wenigstens ein Gas, vorzugsweise Luft eingeleitet wird.

Somit kann das Dialysegerät oder die Zubereitungseinheit Mittel, insbesondere eine oder mehrere Pumpen oder Flüssigkeitsquellen und/oder Kompressoren oder Druckluftquellen aufweisen oder mit diesen in Verbindung stehen oder verbindbar sein, die derart ausgebildet sind, dass durch die Mittel wenigstens eine Flüssigkeit und/oder wenigstens ein Gas in den mit dem Dialysegerät oder der Zubereitungseinheit konnektierten Behälter einleitbar ist bzw. eingeleitet wird.

Des Weiteren kann über diese Mittel, insbesondere eine oder mehrere Pumpen, die in dem mit dem Dialysegerät oder der Zubereitungseinheit konnektierten Behälter befindliche Lösung bzw. das saure Flüssigkonzentrat aus dem Behälter abgeführt werden. Mittels der Mittel kann das saure Flüssigkonzentrat beispielsweise einer Flüssigkeit, z.B. Wasser oder einer Mischung aus wasser und einem basischen Konzentrat zudosiert werden, die in einer Hauptleitung strömt, um auf diese Weise ggf. nach Zugabe eines basischen Konzentrats eine fertige Dialyselösung herstellen zu können.

Das Dialysegerät oder die Zubereitungseinheit kann wenigstens eine Dosiereinrichtung aufweisen, die mit den genannten Mitteln in Verbindung steht oder diese umfasst, wobei die Dosiereinrichtung derart ausgebildet ist, dass sie das aus dem Behälter abgeführte saure Flüssigkonzentrat in einen Flüssigkeitsstrom oder in ein vorzugsweise teilweise oder vollständig mit Flüssigkeit, vorzugsweise mit Wasser gefülltes Behältnis zum Zwecke der Herstellung der Dialyselösung zudosiert.

Weiterhin kann vorgesehen sein, dass das Dialysegerät oder die Zubereitungseinheit wenigstens eine Hauptleitung und wenigstens eine Nebenleitung aufweist, die in die Hauptleitung mündet und in oder an der der wenigstens eine erfindungsgemäße Behälter angeordnet ist. So ist es beispielsweise denkbar, dass der wenigstens eine Behälter in einer Nebenleitung angeordnet ist, die an wenigstens einer oder auch an zwei Stellen mit einer Hauptleitung in Verbindung steht. Im Falle von zwei Nebenleitungen ist es denkbar, dass durch eine zu dem Behältnis führende Nebenleitung Wasser oder Wasser und Luft dem Behälter zugeführt wird und dass durch eine von dem Behältnis abführende Leitung das flüssige Konzentrat aus dem Behältnis abgeführt wird.

Ebenso ist es denkbar, dass die Nebenleitung nur an einer Stelle mit einer Hauptleitung in Verbindung steht und der Lösevorgang derart ausgestaltet ist, dass von der Hauptleitung eine Flüssigkeit, vorzugsweise RO-Wasser und/oder Gas in den Behälter strömt, und nach dem Auflösen des Trockenkonzentrats über dieselbe Nebenleitung das flüssige Konzentrat sodann wieder in die Hauptleitung oder in eine andere Leitung oder in ein Behältnis geführt wird.

Weiterhin kann wenigstens eine Steuer- oder Regeleinheit vorgesehen sein, die derart ausgeführt ist, dass sie die Zufuhr von Flüssigkeit, vorzugsweise Wasser, oder von Flüssigkeit, vorzugsweise Wasser, und Gas, vorzugsweise Luft, in den Behälter und/oder die Abfuhr von Gas, vorzugsweise Luft und/oder des sauren Flüssigkonzentrats aus dem Behälter in Abhängigkeit von der Zeit und/oder nach einem bestimmten Programm steuert oder regelt und/oder in einer vorbestimmten Menge zu- bzw. abführt.

Vorzugsweise wird der Lösevorgang so durchgeführt, dass in dem Behälter kein oder kein wesentlicher Überdruck besteht. Vorzugsweise wird zur Atmosphäre hin belüftet, das heißt der Behälter steht unmittelbar oder mittelbar über das Gerät bzw. über die Zubereitungseinheit mit der Atmosphäre in Verbindung.

Auch ist es möglich, dass die Steuer- oder Regeleinheit derart ausgebildet ist, dass sie die Zufuhr von Flüssigkeit und/oder Gas in den Behälter und/oder die Abfuhr der Lösung aus dem Behälter in Abhängigkeit von der Zeit, d. h. beispielsweise für eine bestimmte Dauer oder gemäß einem bestimmten Zeitprogramm steuert oder regelt und/oder dass dem Behältnis eine bestimmte Menge an Wasser zugegeben wird, um die gewünschten Konzentrationen der Stoffe zu erhalten.

So ist es beispielsweise denkbar, dass Flüssigkeit, insbesondere Wasser, oder Flüssigkeit, insbesondere Wasser, und Gas, insbesondere Luft, für eine vorbestimmte Zeit oder in vorbestimmten Intervallen und/oder in einem bestimmten Volumen und/oder Masse dem Behälter zugeführt wird.

Vorzugsweise erfolgt die Herstellung des sauren Konzentrats nicht kontinuierlich, sondern batchweise. Zunächst wird vorzugsweise Luft und Wasser in das Trockenkonzentrat bzw. in den Behälter eingeführt und überschüssige Luft wieder abgezogen. Vorzugsweise ist weiter vorgesehen, dass nach der vollständigen Auflösung des Trockenkonzentrats das flüssige Konzentrat aus dem Behälter über eine Pumpe oder dergleichen abgeführt wird.

Vorzugsweise ist vorgesehen, dass die Rezeptur eine gewisse Menge Wasser vorschreibt. Diese Menge Wasser wird dann beispielsweise in diskreten Portionen oder kontinuierlich durchgeführt. Denkbar ist beispielsweise eine Wasserzudosierung in Form von mehreren 30 ml Volumina, die nacheinander zugegeben werden.

Weiterhin kann vorgesehen sein, dass die Steuer- oder Regeleinheit derart ausgebildet ist, dass die Zufuhr von Flüssigkeit oder von Flüssigkeit und Gas kontinuierlich, intervallweise oder batchweise erfolgt. Denkbar ist es beispielsweise einmal eine bestimmte Menge an Flüssigkeit bzw. Wasser zuzugeben und kontinuierlich oder einmalig oder intermittierend oder permanent ein Gas, insbesondere Luft in den Behälter einzuführen, um den Lösevorgang zu beschleunigen.

Außerdem betrifft die vorliegende Erfindung eine weitere Ausgestaltung eines Dialysegerätes oder einer Zubereitungseinheit. Dabei ist vorgesehen, dass ein Dialysegerät oder eine Zubereitungseinheit, wobei die Zubereitungseinheit zur Herstellung eines Konzentrats zur Herstellung einer Dialyselösung dient, derart ausgeführt wird, dass das Dialysegerät oder die Zubereitungseinheit wenigstens ein Verbindungsmittel aufweist, mittels dessen ein Druckluftleitungsmittel mit einem Flüssigkeitsleitungsmittel verbindbar oder verbunden ist, wobei mittels des Verbindungsmittels Flüssigkeit oder Flüssigkeit und Gas zu einem Behälter enthaltend wenigstens ein Trockenkonzentrat zuführbar ist und wobei mittels des Verbindungsmittels saures Flüssigkonzentrat aus dem Behälter ableitbar ist.

Es ist möglich, dass wenigstens eine Steuer- oder Regeleinheit vorgesehen ist, die derart ausgeführt ist, dass sie die Zufuhr von Flüssigkeit, vorzugsweise Wasser, oder Flüssigkeit, vorzugsweise Wasser, und Gas, vorzugsweise Luft, in den Behälter und/oder die Abfuhr von Gas, vorzugsweise Luft und/oder des sauren Flüssigkonzentrats aus dem Behälter steuert oder regelt und/oder dass das Dialysegerät oder die Zubereitungseinheit weiter die kennzeichnenden Merkmale gemäß einem der Ansprüche 7 bis 9 aufweist. Insbesondere kann es sich bei dem Behälter um einen Behälter gemäß einem der Ansprüche 1 bis 4 und/oder gemäß vorstehender Beschreibung handeln. Denkbar ist auch, dass das Dialysegerät oder die Zubereitungseinheit zur Konnektion mit einem Behälter gemäß einem der Ansprüche 1 bis 4 und/oder gemäß vorstehender Beschreibung geeignet ist. Die Flüssigkeit kann Wasser, insbesondere RO-Wasser sein und das Gas kann insbesondere Luft sein.

Denkbar ist also insbesondere, dass bei einem Dialysegerät oder einer Zubereitungseinheit eine Verbindung von einer Druckluftleitung zur einer Wasserleitung vorgesehen ist, die zur Zufuhr von RO-Wasser in den Behälter und zur Ableitung des Konzentrates dient. Zusätzlich weist dieses Dialysegerät oder diese Zubereitungseinheit eine Steuer- und/oder Regelungseinheit mit einem Programm für die gleichzeitige Einführung von Luft und Wasser auf.

Die vorliegende Erfindung betrifft des Weiteren ein Verfahren zur Herstellung eines sauren Flüssigkonzentrates, das zur Herstellung einer Dialyselösung dient, wobei das Verfahren die folgenden Schritte umfasst: Konnektieren eines erfindungsgemäßen Behälters an ein Dialysegerät oder an eine Zubereitungseinheit, vollständiges Auflösen des Trockenkonzentrats und Abfuhr der durch das vollständige Auflösen des Trockenkonzentrats erhaltenen sauren Flüssigkonzentrats aus dem Behälter.

Denkbar ist es, wenn zum Zwecke des Auflösens des Trockenkonzentrats in den Behälter eine Flüssigkeit, vorzugsweise Wasser, oder eine Flüssigkeit, vorzugsweise Wasser, und Gas, vorzugsweise Luft, eingeleitet wird.

Weiterhin kann vorgesehen sein, dass zum Zwecke des Auflösens des Trockenkonzentrats in den Behälter zunächst eine Flüssigkeit, vorzugsweise Wasser, und anschließend Gas, vorzugsweise Luft, oder Flüssigkeit, vorzugsweise Wasser, und Gas, vorzugsweise Luft, gleichzeitig eingeleitet werden.

Es ist möglich, dass die Flüssigkeit, vorzugsweise Wasser, oder die Flüssigkeit, vorzugsweise Wasser, und das Gas, vorzugsweise Luft, für eine bestimmte Zeitspanne und/oder in einem bestimmten Volumen oder Masse und/oder durch eine bestimmte Anzahl der Förderbewegungen und/oder durch eine bestimmte Betriebsdauer einer Fördereinheit gefördert wird. Die Flüssigkeit oder die Flüssigkeit und das Gas kann/können also für eine bestimmte Zeitspanne, durch eine bestimmte Anzahl der Förderbewegungen einer Fördereinheit, wie beispielsweise einer Pumpe, in den Behälter gefördert werden. Denkbar ist es dass beispielsweise eine bestimmte Anzahl von Bilanzkammerfüllungen des Dialysegerätes verwendet wird, um den erfindungsgemäßen Behälter mit einer Flüssigkeit bzw. Wasser zu füllen.

Bei der Verwendung der Bilanzkammer eines Dialysegerätes als Dosiersystem zur Zugabe des Lösungsmittels und/oder zur Abfuhr des flüssigen Konzentrats können nicht beliebige Volumina, sondern nur ganzzahlige Vielfache des Bilanzkammervolumens zugegeben werden. Um bei einer vorgegebenen Salzmenge zu der richtigen Konzentration der fertigen Dialyselösung zu gelangen, werden die bei der Auflösung erhaltenen Konzentrationen bei der Verdünnung des Konzentrats berücksichtigt. Ein ähnliches Verfahren ist aus der EP 0 548 537 A2 bekannt, auf die hier insoweit Bezug genommen wird.

Beträgt beispielsweise das Sollvolumen der Bilanz- bzw. Dosierkammer 30 ml, kann aufgrund einer Toleranz im Bereich von - 1 ml bis + 2 ml das exakte Volumen der Kammer abweichen. Dieses exakte Volumen der Bilanzkammer kann exemplarisch 29,50 ml betragen. Dieser Wert liegt im Dialysegerät vor.

Schreibt nun die Rezeptur beispielsweise eine Wassermenge von 4500 ml vor, ergeben sich aus 4500 ml : 29,5 ml = 152,542 Umschaltungen des Dosiersystems. Dies entspricht 152 Umschaltungen und einem Rest von 29,5 ml * 0,542 = 15,99 ml. Diese 15,99 ml werden bei der Berechnung des tatsächlichen Mischungsverhältnisses berücksichtigt.

Aus dem auf diese Weise berechneten Mischungsverhältnis im Beutel bzw. im Behältnis wird dann das Pumpvolumen der Konzentratpumpe berechnet und dieser mitgeteilt.

Die vorgenannten Werte sind seibstverständiich nur exemplarischer Natur und soilen nur die generelle Vorgehensweise verdeutlichen, wie sie bei der Verwendung des Bilanzkammersystems als Dosiermittel verwendet werden kann. Diese Vorgehensweise ist ebenfalls Bestandteil der vorliegenden Erfindung.

Weiterhin kann vorgesehen sein, dass die Transmission oder die Absorption der in dem Behälter befindlichen Lösung gemessen wird und in Abhängigkeit davon bestimmt wird, in welchem Umfang das Trockenkonzentrat aufgelöst ist. Erreicht beispielsweise die Transmission einen bestimmten Wert, kann daraus geschlossen werden, dass das Trockenkonzentrat vollständig aufgelöst ist. Sodann kann das auf diese Weise gebildete saure Flüssigkonzentrat aus dem Behälter abgeführt werden und zur Herstellung einer Dialyselösung verwendet werden.

Denkbar ist es, dass die Flüssigkeit oder die Flüssigkeit und das Gas kontinuierlich, intervallweise oder batchweise in den Behälter eingeführt wird und/oder die Lösung aus dem Behälter kontinuierlich, intervallweise oder batchweise abgeführt wird. Es ist vorgesehen, dass eine Abfuhr der Lösung aus dem Behältnis erst dann erfolgt, wenn das Trockenkonzentrat vollständig gelöst ist.

Es ist möglich, dass die Zufuhr von Flüssigkeit, vorzugsweise Wasser, oder von Flüssigkeit, vorzugsweise Wasser, und Gas, vorzugsweise Luft, in den Behälter derart erfolgt, dass das Trockenkonzentrat vollständig in Flüssigkeit, vorzugsweise Wasser, eingetaucht ist, und/oder derart, dass das Trockenkonzentrat durch das Gas, vorzugsweise Luft, bewegt, vorzugsweise aufgewirbelt wird.

Die Menge des zugeführten Wassers ergibt sich beispielsweise aus der Zusammensetzung des Pulvers/Granulats. Eine bestimmte Menge Pulver bzw. Granulat muss mit einer bestimmten Menge Wasser vermischt werden. Mit dieser Menge Wasser muss das Pulver/Granulat völlig aufgelöst werden können. Beim Lösen bzw. beim Befüllen mit Wasser und dem Einbringen der Luft ist das Behältnis vorzugsweise zur Atmosphäre hin offen, und zwar vorzugsweise über den entsprechenden Konnektor des Dialysegerätes bzw. der Zubereitungseinheit.

Bevorzugt ist es, wenn die in dem Behälter befindliche Lösung, d.h. das saure Flüssigkonzentrat erst dann aus dem Behälter abgeführt wird, wenn das in dem Behälter befindliche Trockenkonzentrat vollständig aufgelöst ist. Anzustreben ist eine vollständige Auflösung des Trockenkonzentrats nicht nur, um das Material des Trockenkonzentrats optimal zu verwerten, sondern auch um eine reproduzierbare Zusammensetzung des sauren Konzentrats bereitzustellen.

Weitere Einzelheiten und Vorteile der Erfindung ergeben sich aus einem in der Zeichnung dargestellten Ausführungsbeispiel. Es zeigen:
- Figur 1:: eine schematische Ansicht eines erfindungsgemäßen Behälters, der zum Teil mit Trockenkonzentrat gefüllt ist,
- Figur 2:: eine perspektivische Ansicht eines erfindungsgemäßen Behältnisses sowie einen Konnektionsbereich eines Dialysegerätes bzw. einer Zubereitungseinheit vor der Konnektion des Behältnisses,
- Figur 3:: eine schematische Ansicht einer Ausführungsform des erfindungsgemäßen Behälters bei der Herstellung sowie während des Öffnungsvorgangs,
- Figur 4:: eine Längsschnittansicht durch einen erfindungsgemäßen Behälter sowie eine perspektivische Ansicht des erfindungsgemäßen Behälters,
- Figur 5:: eine Draufsicht auf die Folienbahn zur Herstellung des Behälters,
- Figur 6, 7:: schematische Darstellungen des Herstellprozesses.

Der Behälter 10 ist als Beutel 10 ausgeführt, der flexible Wandungen 14 aufweist. Der Beutel 10 bzw. dessen Wandungen können aus einem Kunststoff bestehen, der vorzugsweise transparent ausgeführt ist.

Die folgenden Ausführungen sind nicht auf einen Beutel beschränkt, sondern können ebenso für jedes beliebige andere Behältnis, wie z.B. eine Kartusche mit starren Wandungen gelten.

In dem in Betriebsposition unteren Bereich des Beutels 10 befinden sich zwei Wandungsabschnitte 17, 18, die beide gegenüber der Horizontalen schräg ausgeführt sind und jeweils von außen nach innen fallend ausgebildet sind und zwischen sich einen talförmigen Bereich 19 bilden, der den untersten Punkt oder Bereich bildet, in dem sich das Trockenkonzentrat 20 in dem Behälterinnenraum befindet.

Wie dies aus der Figur weiter hervorgeht, ist der Behälter 10 nicht vollständig mit Trockenkonzentrat 20 gefüllt, sondern nur zu einem Teil.

In dem dargestellten Ausführungsbeispiel ist der Behälter 10 weniger als zur Hälfte mit Trockenkonzentrat 20 gefüllt. Oberhalb dieser Schüttung befindet sich Luft oder ein sonstiges Gas.

Das Trockenkonzentrat umfasst die folgenden Substanzen: Elektrolyte, Glucose, und Zitronensäure oder eine andere geeignete Säure in fester oder flüssiger Form.

Die genannten Elektrolyte können aus der Gruppe NaCl, KCl, CaCl₂, MgCl₂ ausgewählt sein. Denkbare Mengen bezogen auf einen Liter der fertigen Dialyselösung können im Bereich von 130 - 150 mmol NaCl, 1 - 4 mmol KCl, 1 - 1,75 mmol CaCl₂ und 0,5 - 1 mmol MgCl₂ liegen. Für Zitronensäure kann dieser Wert im Bereich von 1 mmol/l - 15 mmol/l liegen.

Glucose kann in dem Trockenkonzentrat in einer Menge derart vorliegen, dass sich in der gebrauchsfertigen Dialyselösung eine Glucose-Konzentration im Bereich von 1 -2 mmol/l ergibt.

Eine mögliche Vorgehensweise besteht darin, die genannten Komponenten einzeln einzuwiegen und aus allen Komponenten ein Granulat zu bilden, das dann als Trockenkonzentrat 20 in den Behälter bzw. Beutel 10 eingefüllt wird.

Der Beutel 10 kann ein Volumen bzw. eine Aufnahmekapazität von ca. 4 - 15 Litern des sauren Flüssigkonzentrats aufweisen, das durch die Lösung des Trockenkonzentrats gebildet wird. Diese Menge kann für 1 - 3 Hämodialyse-Behandlungen ausreichen.

Der Beutel 10 weist in seinem in der Figur oben dargestellten Bereich ein spezielles Verbindungselement 12 auf, mit dem der Beutel an ein Dialysegerät bzw. an eine Füllstation angehängt werden kann, die im Rahmen der vorliegenden Erfindung auch als Zubereitungseinheit bezeichnet wird. Das Verbindungselement 12 kann derart ausgebildet sein, dass er mit einem speziellen Konnektor des Dialysegerätes oder einer Zubereitungseinheit vorzugsweise fluiddicht und/oder gasdicht verbindbar ist. Diese Verbindung kann beispielsweise durch einfaches Aufstecken oder durch eine Drehbewegung bzw. Schraubverbindung hergestellt werden.

Nach Herstellung dieser Verbindung kann mittels der Leitung 16 Flüssigkeit und/oder Gas in den Beutel 10 eingeleitet werden. Ist das Trockenkonzentrat vollständig aufgelöst, kann das saure flüssige Konzentrat ebenfalls mittels der Leitung 16 aus dem Beutel 10 abgesaugt werden. Die Leitung 16 ragt ausgehend von einer oberen Behälterwandung von oben in das Innere des Behälters und bis in die tiefste Stelle des Innenraums des Behältnisses 10 bzw. bis in das dort vorliegende Trockenkonzentrat.

Das trockene salzhaltige Konzentrat kann in dem Beutel 10 beispielsweise in einer Menge von 1,3 - 4,2 kg vorliegen.

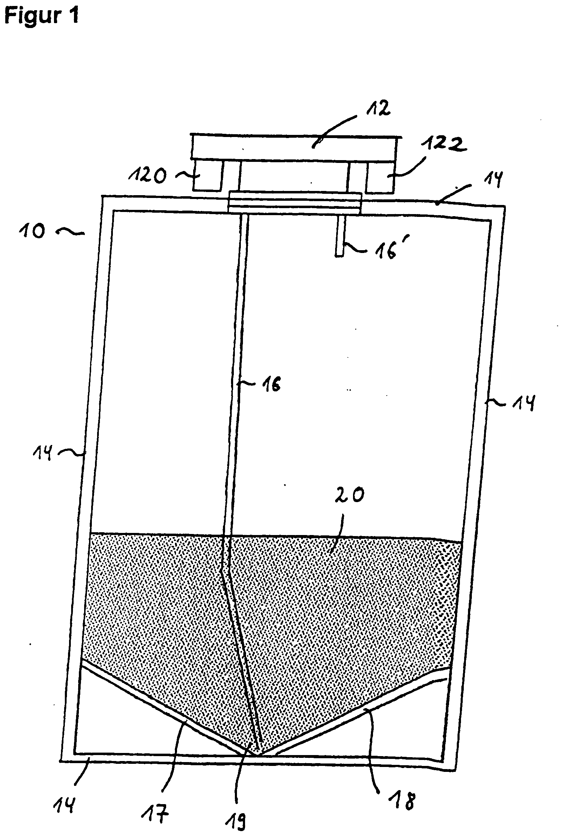

Wie dies weiter aus der Figur 1 hervorgeht und wie dies oben bereits erwähnt wurde, weist der Beutel einen Zulauf 16 auf, der in Form eines Schlauches 16 ausgebildet ist und dessen offenes Ende in dem oben genannten tiefsten Punkt 19 des Beutels 10 liegt.

Der Zulauf bzw. der Schlauch 16 ist in seinem in den Behälter ragenden Endbereich als Schutz mit einer Kappe, Stopfen, einer Membran oder dergleichen versehen, um das Eindringen des Trockenkonzentrats in das Innere des Schlauches 16 zu verhindern. Das spezifische Gewicht dieses Verschlusses liegt vorzugsweise im Bereich < 9,7 kN/m³.

Durch die Leitung 16 erfolgt das Befüllen mit Wasser, das Einbringen von Luft und das Absaugen des flüssigen Konzentrats.

Das Bezugszeichen 16' zeigt ein Element, das als Belüftung zur Atmosphäre hin dient, während des Füllens des Behälters. Nach dem Füllen bzw. nach dem Lösen des Trockenkonzentrats kann diese Belüftung 16' verschlossen sein. Dies bedeutet, dass während der Behandlung dem Element 16' keine Funktion zukommt.

Auch wenn das Element 16' in Figur 1 als kurzes Leitungselement dargestellt ist, ist vorzugsweise vorgesehen, dass keine Leitung 16', sondern nur ein Element, wie beispielsweise ein Filter etc. existiert, über das Luft aus dem Inneren des Behälters 10 entweichen kann. Vorzugsweise weist der Behälter 10 somit nur genau eine Leitung 16 auf, die zur Zufuhr von Wasser und Gas und zur Abfuhr des flüssigen Konzentrats dient.

Beim Entleeren des Behälters im Endstadium der Behandlung wird an beiden Abgängen 16 und 16' gesaugt, um den Behälter möglichst schnell zu entleeren. Anstelle des Elementes 16' oder zusätzlich dazu kann in dem Verbindungsmittel 12 ein Filter sitzen, der verhindert, dass Pulver während des Transports aus dem Beutel heraus gelangt.

Das hergestellte Flüssigkonzentrat kann mit einem Volumen von Wasser, vorzugsweise RO-Wasser gemischt werden, um die fertige Dialyselösung herstellen zu können. Das basische Flüssigkonzentrat, das ebenfalls durch Lösung eines Trockenkonzentrats in einem mit dem Dialysegerät oder einer Zubereitungseinheit konnektierten Behälter erhalten werden kann, kann vor oder nach der Zugabe des sauren Flüssigkonzentrats zudosiert werden.

Grundsätzlich ist von der Erfindung umfasst, dass der das basische Konzentrat enthaltende Behälter räumlich getrennt von dem Behälter angeordnet ist, der das saure Trockenkonzentrat enthält. Jedoch ist von der Erfindung auch der Fall umfasst, dass beide Behälter miteinander in Verbindung stehen und ggf. unterschiedliche Kompartimente oder Kammern eines gemeinsamen Behälters bzw. Beutels bilden.

Um das Entweichen von Luft aus dem Behälter bei dessen Befüllung mit Wasser und/oder Luft zu ermöglichen, ist - wie ausgeführt - ein Entlüftungselement vorgesehen, das mit dem Dialysegerät oder mit der Zubereitungseinheit in Verbindung stehen kann. Dieses kann durch einen Filter gebildet werden, wobei dieser vorzugsweise in der Beutelwandung oder im Bereich des Verbindungsmittels 12 angeordnet ist. Dieser Filter sollte derart ausgeführt sein, dass er Luft und Flüssigkeit hindurchlässt, jedoch kein Pulver bzw. Granulat, so dass dieses nicht unbeabsichtigt aus dem Behältnis gelangen kann.

Um eine eindeutige Identifizierung des Beutels vornehmen zu können, ist es denkbar, dass dieser mit einer Codierung, wie z.B. einem Barcode, 2-Matrix-Code oder RFID ausgebildet ist. Denkbar ist es, dass das Dialysegerät und/oder eine Zubereitungseinheit zur Herstellung des sauren Flüssigkonzentrats eine Leseeinrichtung aufweist, mittels derer diese Codierung erfasst bzw. ausgelesen werden kann. Basierend auf dieser Information kann dann vorzugsweise selbsttätig durch das Dialysegerät bzw. durch die Zubereitungseinheit ein Verfahren zum Lösen des Trockenkonzentrats 20 in dem Beutel 10 eingeleitet werden.

Dabei ist es denkbar, dass die Details des Verfahrens von der genannten Beutel- bzw. Behältercodierung abhängen. Dies kann beispielsweise für die Menge des zugegebenen Wassers oder des zugegebenen Wassers und der Luft zum Lösen des Trockenkonzentrats, für die Dauer des Lösevorgangs, für die Frage, ob der Lösevorgang durch die Zufuhr von Luft unterstützt wird, für die Reihenfolge der Zugabe von Luft und Wasser, für die Frage, ob ein kontinuierlicher, ein intermittierender oder ein einmaliger (batchweiser) Lösevorgang erfolgt, etc. gelten.

Aufgrund der Codierung können ferner Leitfähigkeitsgrenzwerte gesetzt werden.

Ein möglicher Ablauf des Löseprozesses kann sich beispielsweise derart gestalten, dass zunächst in den Beutel 10 Wasser, vorzugsweise RO-Wasser bei einer Wassertemperatur von 37 °C bis 85 °C durch den Schlauch 16 zugegeben wird.

Der Wasserfluss kann in einem Bereich bis 1200 ml/min liegen.

In einer zweiten anschließenden Phase kann dann vorzugsweise ebenfalls über die Leitung 16 Luft für einen bestimmten Zeitraum in den Beutel 10 eingeleitet werden.

Alternativ dazu kann die Einleitung von Wasser und Luft in den Behälter zeitgleich erfolgen.

Auch ist es möglich, Luft gleichzeitig mit dem Wasser in den Behälter einzuführen, dann den Wasserzulauf abzustellen und die Luftzufuhr fortzusetzen, vorzugsweise bis zur vollständigen Auflösung des Salzes bzw. des Trockenkonzentrates 20.

In den Beutel 10 bzw. Behälter 10 kann generell soviel Wasser oder eine sonstige Flüssigkeit gegeben werden, dass dieser vollständig oder nur teilweise gefüllt wird. Die Füllung des Beutels 10 mit Wasser kann beispielsweise durch eine ganzzahlige Anzahl von Bilanzkammerfüllungen der Bilanzkammer/Dosierkammer eines Dialysegerätes erfolgen. Das tatsächliche Füllvolumen des Beutels 10 kann dann mit Hilfe des bekannten Bilanzkammervolumens ermittelt werden. Die Konzentrationen der einzelnen Stoffe ergeben sich aus dem Verhältnis von Pulver bzw. Granulat zu Wasser. Zur Füllung des Beutels mit Wasser kann auch jede andere Dosiermöglichkeit, wie beispielsweise eine Pumpe etc. zum Einsatz kommen.

Die Dauer der Zufuhr des Gases, das vorzugsweise als gereinigte bzw. filtrierte Luft ausgeführt ist, kann in Abhängigkeit vom Füllvolumen und/oder vom Luftfluss abhängen.

In einer Ausgestaltung der Erfindung ist ein Timer vorgesehen, der die Füllung bzw. Aufbereitung des sauren Flüssigkonzentrats nach Ende der letzten Dialysebehandlung vornimmt (AutoOFF) oder vor Beginn der Dialyse (AutoON). Auch der Einsatz eines Timers für die Zubereitungseinheit, die auch als Füllstation bezeichnet wird, ist möglich.

Werden mit demselben Beutel 10 mehrere Dialysebehandlungen nacheinander durchgeführt, ist es möglich, eine Zwischendesinfektion zwischen den Behandlungen durchzuführen. Denkbar ist es, vor dem Freispülen zu Füllen, wobei kein Kontaminationsrisiko besteht, da es sich um den Primärkreislauf handelt.

Des Weiteren ist eine "Reichweitenbestimmung", d.h. eine Bestimmung der möglichen Nutzungsdauer zur optimalen Nutzung des sauren Flüssigkonzentrates möglich und von der Erfindung mit umfasst.

Anstelle eines Kanisters mit flüssigem Säurekonzentrat insbesondere für die Bicarbonatdialyse wird erfindungsgemäß ein Beutel oder sonstiger Behälter mit einem trockenen Salzkonzentrat eingesetzt. Dieser Beutel oder Behälter wird am Dialysegerät oder an einer Füllstation vorzugsweise mit einer definierten Menge von Wasser für die Dialyse gefüllt und vorzugsweise durch Einbringen von sterilfiltrierter Luft aufgelöst. Nach dem Auflösungsprozess steht ein Säurekonzentrat zur Verfügung, das in bevorzugter Ausgestaltung der Erfindung den Anforderungen gemäß EN 13867:2002 entspricht.

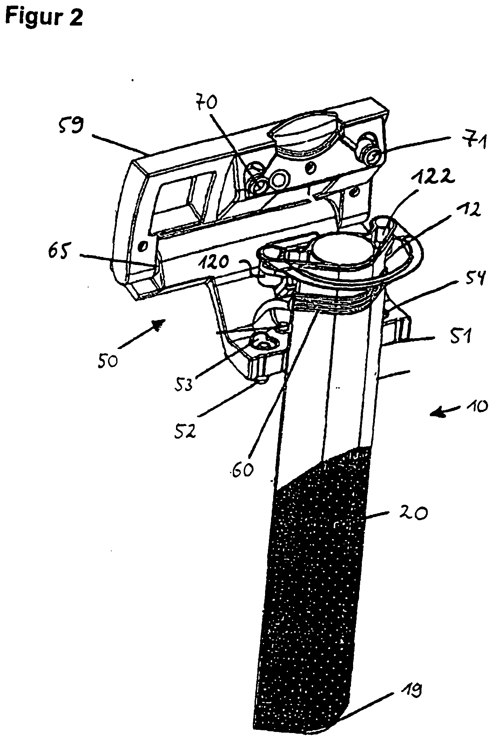

Figur 2 zeigt eine exemplarische Ausgestaltung des erfindungsgemäßen Behälters mit dem Verbindungsmittel 12, das zur Konnektion an das Konnektionsmittel einer Zubereitungseinheit oder eines Dialysegerätes dient.

Die aus Figur 2 ersichtliche Anordnung stellt in bevorzugter Ausgestaltung eine erfindungsgemäße Anordnung zur Konnektion eines Behälters 10 an das Dialysegerät bzw. an eine Zubereitungseinheit dar, die allgemein mit dem Bezugszeichen 50 gekennzeichnet ist.

Durch den Anschluss 52 des Geräts bzw. der Zubereitungseinheit kann Luft und Wasser durch die Verbindungsmittel 12 zum Behälter 10 und durch den Schlauch 16 in den unteren Bereich des Behälters eingeleitet werden. Dazu erfolgt eine Verbindung des Anschlusses 52 mit dem behälterseitigen Verbinder 120.

Des Weiteren steht der Behälter 10 bzw. dessen Verbindungsmittel 12 über einen weiteren Konnektor bzw. Verbinder 122 mit der Dialysemaschine bzw. der Zubereitungseinheit in Verbindung. Die entsprechend zugehörige Leitung ist mit dem Bezugszeichen 51 markiert.

Diese steht mit dem Element 16' des Behältnisses in Verbindung.

Durch den Port bzw. die Leitung 51 wird während des Lösevorgangs bzw. während des Befüllvorgangs des Behälters mit Wasser und Luft, Luft aus dem Behälter abgezogen.

Abweichend von der aus der EP 1 344 550 B1 bekannten Lehre dient die Leitung 52 bzw. die Leitung 16 gemäß der vorliegenden Erfindung nicht nur zur Zufuhr von Wasser und Luft sondern auch zur Abfuhr des gelösten Konzentrats. Dieses wird mittels geeigneter Fördermittel, wie beispielsweise mittels einer Pumpe durch die Leitung 16 und die Leitung 52 abgezogen und dann an geeigneter Stelle im Dialysegerät bzw. in der Zubereitungseinheit verdünnt, so dass gegebenenfalls nach Zugabe eines basischen Konzentrates eine fertige Dialyselösung bereitgestellt werden kann. Dies bedeutet, dass der Strömungsweg durch das Verbindungsmittel und das Konnektionsmittel sowie durch den Schlauch des zur Lösung verwendeten Wassers zumindest bereichsweise dem entspricht, über den das flüssige Konzentrat abgeführt wird. Auch kann ein und dieselbe Pumpe verwendet werden, um einerseits das Wasser zuzuführen und andererseits das flüssige Konzentrat abzuführen.

Wie dies aus Figur 2 hervorgeht, weisen die Anschlüsse bzw. Leitungen 51, 52 Stutzen auf, auf die die Verbinder 120, 122 des Behältnisses 10 aufgesetzt und vorzugsweise aufgesteckt werden, so dass Aufnahmebereiche der Verbinder 120, 122 die Stutzen aufnehmen. Dazu sind an dem Gerät bzw. an der Zubereitungseinheit Vertiefungen bzw. Aufnahmen 53, 54 angeordnet, in die die genannten Stutzen ragen und in die die entsprechenden Verbinder 120, 122 eingesetzt werden, wenn das Behältnis 10 konnektiert wird. Im konnektierten Zustand ragen die Stutzen in Aufnahmen der Verbinder 120, 122 oder stehen jedenfalls derart mit diesen in Verbindung, dass eine fluiddichte Verbindung hergestellt ist.

Der Verbinder 120 steht in Fluidverbindung mit dem Schlauch 16 und der Verbinder 122 steht in Fluidverbindung mit dem Element 16' bzw. mit einem Filter des Behältnisses. Eine solche Verbindung kann beispielsweise durch eine Nut oder dergleichen in der Wandung der Verbinder 120, 122 erzielt werden, wie sie in der EP 1 344 550 B1 beschrieben ist. Zwischen dem Verbinder 120, 122 und der Leitung 16 und dem Element 16' kann sich eine Kammer befinden, die ebenfalls in der EP 1 344 550 B1 näher beschrieben ist.

Wie ausgeführt, handelt es sich bei dem Element 16' vorzugsweise nicht um einen Schlauch oder dergleichen, sondern lediglich um ein Mittel, das Luft und ggf. Flüssigkeit hindurchlässt, das trockene Pulver/Granulat zurückhält.

Dementsprechend kann über die gerätseitige Leitung 52 und über den Verbinder 120 Wasser und Luft dem Behälter 10 zugeführt und das flüssige Konzentrat aus dem Behälter 10 abgeführt werden. Über die geräteseitige Leitung 51 und den Verbinder 122 kann Luft aus dem Behälter abgezogen werden bzw. entweichen.

Im eingesetzten Zustand des Behältnisses 10 wird sodann, d.h. nach der Konnektierung des Behältnisses 10 der um die Achse 65 verschwenkbare Deckel 59 des Gerätes bzw. der Zubereitungseinheit heruntergeklappt, so dass die an dem Deckel 59 angeordneten Stutzen 70, 71 von oben auf die Verbinder 120, 122 des Verbindungsmittels 12 drücken und diese in der konnektierten Position halten. Ist kein Behältnis eingesetzt, können die Stutzen 70, 71 in die Ausnehmungen 53, 54 eingreifen, so dass sich ebenfalls eine fluiddichte Verbindung ergibt. In dieser Position des Deckels kann ein Spülvorgang durchgeführt werden.

Das Bezugszeichen 60 kennzeichnet schließlich den Endbereich des Beutels 10, der mit dem Verbindungselement 12 dicht verbunden ist.

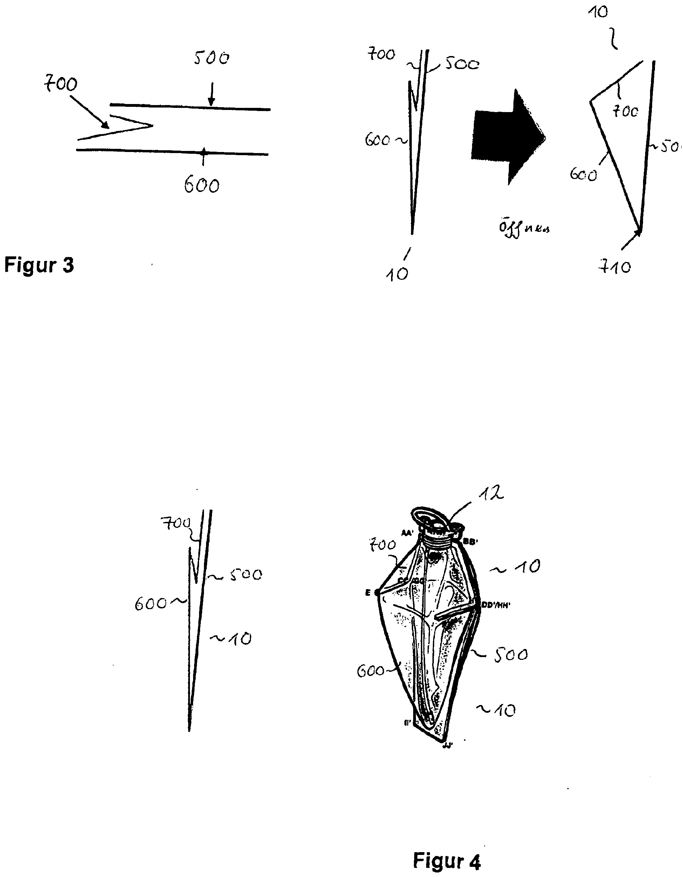

Figur 3 zeigt mit dem Bezugszeichen 500 eine erste Folie und mit dem Bezugszeichen 600 eine zweite Folie einer Ausführungsform des Behälters 10. Mit dem Bezugszeichen 700 ist ein gefalteter Bereich gekennzeichnet, der die beiden Folien 500 und 600 verbindet. Dieser gefaltete Bereich 700 kann als separates Teil ausgeführt sein oder einteilig mit einer der Folien 500, 600 in Verbindung stehen. Wie dies aus Figur 3 ersichtlich ist, wird der gefaltete Bereich 700 durch zwei Schenkel gebildet, die spitz zueinander angeordnet sind, jedoch nicht die gleiche Länge haben.

Figur 3 zeigt des Weiteren den Öffnungsprozess des Benälters von dem iinks von dem Pfeil dargestellten zusammengefalteten Zustand und in dem rechts von dem Pfeil dargestellten ausgefalteten Zustand.

Durch die ungleich langen Schenkel des Falzbereiches 700 wird erreicht, dass beim Auffalten der Schwerpunkt sich weniger stark bewegt als bei gleich langen Schenkeln, so dass die mit dem Bezugszeichen 710 dargestellte V-förmige Spitze des Behälters unten verbleibt, was für den Lösevorgang von Bedeutung ist.

Der im Rahmen der vorliegenden Erfindung verwendete Begriff "Folie" ist allgemein zu fassen und umfasst jedes beliebige Wandungsmaterial des Behälters. Dieses kann elastisch, flexibel etc. ausgebildet sein.

Die Folien 500, 600 und der Falz 700 bzw. der Eckbereich 700 können aus demselben Material bestehen.

Figur 4, linke Darstellung zeigt den Behälter gemäß Figur 3 in einer Längsschnittdarstellung sowie in einer perspektivischen Ansicht. Im oberen Bereich des Behälters ist das Verbindungsmittel 12 angeordnet, mittels dessen der Behälter an ein Dialysegerät oder an eine Zubereitungseinheit für das saure Konzentrat konnektierbar ist. Dieses Verbindungsmittel steht fluiddicht mit den angrenzenden Folien bzw. Wandungsbereichen des Behälters 10 in Verbindung.

Wie dies aus Figur 4 hervorgeht, wird eine Wandung des Behälters durch eine Folie gebildet und die andere Wandung des Behälters durch die andere Folie, die den falzförmigen Abschnitt aufweist. Aus den Figuren geht hervor, dass der Behälter im eingefalteten Zustand relativ wenig Raum in Anspruch nimmt, im ausgefalteten Zustand jedoch ein großes Aufnahmevolumen für das Trockenkonzentrat bzw. für das gelöste Konzentrat aufweist.

In einer bevorzugten Ausgestaltung der Erfindung ist vorgesehen, dass der Behälter bzw. dessen Wandungen genau aus zwei Folien hergestellt wird.

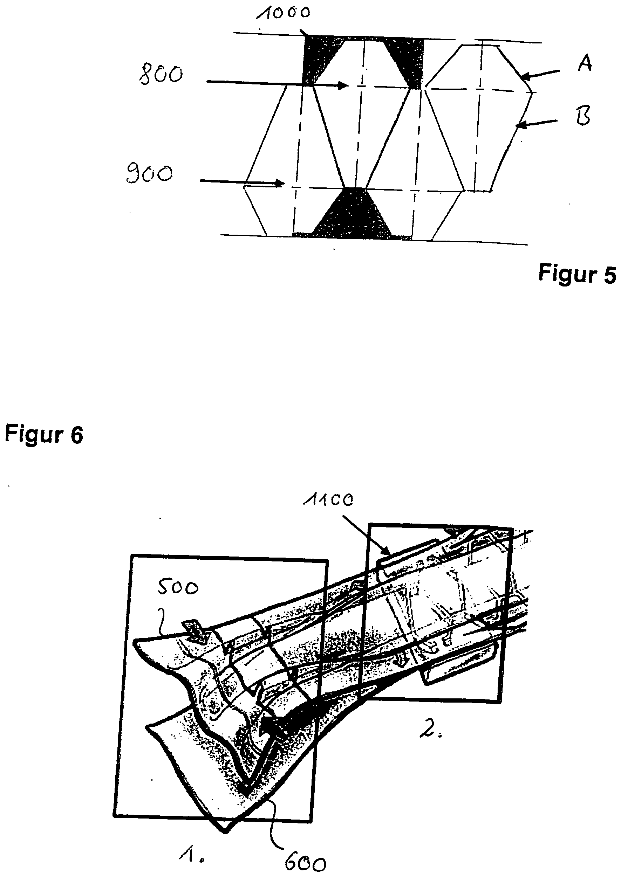

Figur 5 zeigt in einer Draufsicht die Folienbahn, die aus zwei Reihen 800, 900 besteht. Wie dies aus der Figur hervorgeht, sind die Folienabschnitte zur Bildung der Behälterwandungen in der Draufsicht trapezförmig ausgeführt und so in den beiden Bahnen angeordnet, dass die untere Reihe 900 der trapezförmigen Abschnitte gegenüber der oberen Reihe 800 der trapezförmigen Abschnitte gestürzt ist, d. h. auf dem Kopf steht. Dies erlaubt eine gute Ausnutzung des Folienmaterials.

Wie dies weiter aus Figur 5 hervorgeht, ist der Verschnitt 1000 auf diese Weise relativ gering gehalten und befindet sich vorzugsweise nicht zwischen den aneinander angrenzenden Trapezen.

Wie dies durch das Bezugszeichen A gekennzeichnet ist, wird das Verschweißen in einem Schritt entsprechend des dargestellten Muster M erzeugt und das Schneiden der Folien in einem darauf folgenden Schritt B ebenfalls entsprechend des Musters M.

Figur 6 zeigt in einer perspektivischen Darstellung die Folien 500, 600, die den Behälter ausbilden. Wie dies aus Figur 5 ersichtlich und durch Pfeile angedeutet ist, wird in Schritt 1 zunächst die obere der Folien 500 gefaltet und dann in Schritt 2 mit der unteren Folienbahn 600 verschweißt. Der Schnitt des Schneidens der beiden Folien zur Herstellung voneinander getrennter Behälter ist in Figur 5 nicht dargestellt. Das Bezugszeichen 1100 kennzeichnet die Vorrichtung zum Verschweißen der jeweiligen Folienabschnitte.

In bevorzugter Ausgestaltung der Erfindung handelt es sich bei den einzelnen Folien jeweils um mehrschichtige, vorzugsweise um zweischichtige Folien. Eine Schicht stellt eine Abdichtungsschicht dar, die eine geringe Schmelztemperatur aufweist. Die andere Schicht weist eine demgegenüber höhere Schmelztemperatur auf und weist eine relativ dazu gute mechanische Festigkeit bzw. Beständigkeit auf. Denkbar ist der Einsatz von Polyamid (hohe Schmelztemperatur, gute Widerstandseigenschaften, durchsichtig und optisch ansprechend) und Polyethylen (geringere Schmelztemperatur, leicht zu verschweißen). Eine solche zweilagige Folie stellt eine gute Option zur Herstellung der erfindungsgemäßen Behälter dar.

Vorzugsweise liegt die Dicke der Folien bei 200 Mikrometern und die Dimensionierung der Folien ist derart ausgeführt, dass der gefüllte Behälter ein Volumen von 5 Litern aufnehmen kann.

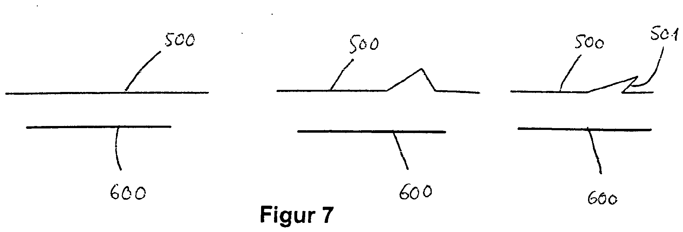

Figur 7 zeigt in der linken Darstellung die beiden Folien 500, 600, die die späteren Wandungen des Behälters ausbilden. In einem ersten Verfahrensschritt werden die Folien abgerollt (Figur 7, linke Darstellung). Sodann wird in eine der Folien 500 eine Falte bzw. ein Knick erzeugt, wie dies aus Figur 7, mittlere und rechte Darstellung hervorgeht. Dieser Knick wird derart ausgeführt, dass die Länge der beiden Folien im eingeknickten Zustand der Folie 500 im Wesentlichen identisch ist. Der durch zwei Schenkel 501 gebildete Bereich bildet eine Wandung des Behälters 10, die weiteren Wandungen werden durch die benachbarten Abschnitte der Folie 500 sowie durch die Folie 600 gebildet.

## Patentansprüche

1. Dialysegerät , **dadurch gekennzeichnet, dass** das Dialysegerät mit einem Behälter (10) konnektiert ist oder zur Konnektion mit einem Behälter (10) geeignet ist, wobei das Dialysegerät Mittel aufweist, die derart ausgebildet sind, dass durch diese Mittel wenigstens eine Flüssigkeit, vorzugsweise Wasser, und wenigstens ein Gas, vorzugsweise Luft, in den mit dem Dialysegerät konnektierten Behälter (10) eingeleitet wird, wobei der Behälter (10) wenigstens ein Trockenkonzentrat (20) enthält, wobei das Trockenkonzentrat (20) derart ausgebildet ist, dass es bei seiner Lösung in einer Flüssigkeit, vorzugsweise in Wasser, wenigstens ein saures Flüssigkonzentrat oder einen Teil eines sauren Flüssigkonzentrats bildet, das zur Herstellung wenigstens einer Dialyselösung geeignet ist, wobei der Behälter (10) derart ausgebildet ist, dass er zumindest ein Verbindungsmittel (12) aufweist, mittels dessen der Behälter (10) an das Dialysegerät konnektierbar ist, wobei der Behälter (10) wenigstens ein Einführmittel (16) aufweist, durch das zum Zwecke des Lösens des Trockenkonzentrats (20) die Flüssigkeit und das Gas in den Behälter (10) einleitbar ist, wobei das Einführmittel (16) als Schlauch ausgebildet ist oder zumindest einen Schlauch umfasst, wobei weiter vorgesehen ist, dass das Einführmittel (16) derart dimensioniert ist, dass es bis in das Trockenkonzentrat (20) hinein ragt.

2. Dialysegerät nach Anspruch 1, **dadurch gekennzeichnet, dass** das Dialysegerät Mittel aufweist, die derart ausgebildet sind, dass durch diese Mittel das in dem mit dem Dialysegerät konnektierten Behälter (10) befindliche saure Flüssigkonzentrat aus dem Behälter (10) abführbar ist oder abgeführt wird, wobei vorzugsweise vorgesehen ist, dass es sich bei diesen Mitteln teilweise oder vollständig um dieselben Mittel handelt, durch die wenigstens eine Flüssigkeit, vorzugsweise Wasser, und das wenigstens eine Gas, vorzugsweise Luft, in den mit dem Dialysegerät konnektierten Behälter (10) einleitbar ist bzw. eingeleitet wird.

3. Dialysegerät nach Anspruch 1 oder 2, **dadurch gekennzeichnet, dass** wenigstens eine Steuer- oder Regeleinheit vorgesehen ist, die derart ausgeführt ist, dass sie die Zufuhr der Flüssigkeit und des Gases in den Behälter (10) und/oder die Abfuhr von Gas, vorzugsweise Luft und/oder des sauren Flüssigkonzentrats aus dem Behälter (10) steuert oder regelt.

4. Dialysegerät **dadurch gekennzeichnet, dass** das Dialysegerät wenigstens ein Verbindungsmittel (16, 52) aufweist, mittels dessen ein Druckluftleitungsmittel mit einem Flüssigkeitsleitungsmittel verbunden ist, wobei mittels des Verbindungsmittels (16, 52) Flüssigkeit und Gas zu einem Behälter (10) enthaltend wenigstens ein Trockenkonzentrat (20) zuführbar ist und wobei mittels des Verbindungsmittels (16, 52) saures Flüssigkonzentrat aus dem Behälter (10) ableitbar ist.

5. Dialysegerät nach Anspruch 4, **dadurch gekennzeichnet, dass** wenigstens eine Steuer- oder Regeleinheit vorgesehen ist, die derart ausgeführt ist, dass sie die Zufuhr von Flüssigkeit, vorzugsweise Wasser, und Gas, vorzugsweise Luft, in den Behälter (10) und/oder die Abfuhr von Gas, vorzugsweise Luft und/oder des sauren Flüssigkonzentrats aus dem Behälter (10) steuert oder regelt und/oder dass das Dialysegerät weiter die kennzeichnenden Merkmale gemäß einem der Ansprüche 1 oder 2 aufweist.

6. Verfahren zur Herstellung eines Flüssigkonzentrates, das zur Herstellung einer Dialyselösung dient, **dadurch gekennzeichnet, dass** es sich um ein saures Flüssigkonzentrat handelt und dass das Verfahren die folgenden Schritte umfasst:
a) Konnektieren eines Behälters (10) an ein Dialysegerät,
b) Auflösen des Trockenkonzentrats (20) in dem Behälter (10),
c) Abfuhr des durch das Auflösen des Trockenkonzentrats (20) erhaltenen sauren Flüssigkonzentrates aus dem Behälter (10), und
d) Einleiten einer Flüssigkeit, vorzugsweise Wasser, und Gas, vorzugsweise Luft in den Behälter (10) zum Zwecke des Auflösens des Trockenkonzentrats (20),
wobei der Behälter (10) wenigstens ein Trockenkonzentrat (20) enthält, wobei das Trockenkonzentrat (20) derart ausgebildet ist, dass es bei seiner Lösung in einer Flüssigkeit, vorzugsweise in Wasser, wenigstens ein saures Flüssigkonzentrat oder einen Teil eines sauren Flüssigkonzentrats bildet, das zur Herstellung wenigstens einer Dialyselösung geeignet ist, wobei der Behälter (10) derart ausgebildet ist, dass er zumindest ein Verbindungsmittel (12) aufweist, mittels dessen der Behälter (10) an das Dialysegerät konnektierbar ist, wobei der Behälter (10) wenigstens ein Einführmittel (16) aufweist, durch das zum Zwecke des Lösens des Trockenkonzentrats (20) die Flüssigkeit und das Gas in den Behälter (10) einleitbar ist, wobei das Einführmittel (16) als Schlauch ausgebildet ist oder zumindest einen Schlauch umfasst, wobei weiter vorgesehen ist, dass das Einführmittel (16) derart dimensioniert ist, dass es bis in das Trockenkonzentrat (20) hinein ragt..

7. Verfahren nach Anspruch 6, **dadurch gekennzeichnet, dass** aus dem Behälter (10) Gas, vorzugsweise Luft abgeführt wird und/oder dass das aus dem Behälter (10) abgeführte saure Flüssigkonzentrat zur Herstellung einer Dialyselösung eingesetzt wird.

8. Verfahren nach Anspruch 6 oder 7, **dadurch gekennzeichnet, dass** das in dem Behälter (10) befindliche saure Flüssigkonzentrat erst dann aus dem Behälter (10) abgeführt wird, wenn das darin befindliche Trockenkonzentrat (20) vollständig aufgelöst ist.

9. Dialysegerät nach einem der Ansprüche 1 bis 3 oder Verfahren nach einem der Ansprüche 6 bis 8, **dadurch gekennzeichnet, dass** der Behälter (10) in wenigstens einem Bereich aufeinander zu gewandte Wandbereiche (17, 18) aufweist, zwischen denen sich ein talförmiger Bereich (19) oder eine Vertiefung ausbildet, wobei das Trockenkonzentrat (20) in der Betriebsposition des Behälters (10) zumindest auch in dem talförmigen Bereich (19) bzw. in der Vertiefung vorliegt.

10. Dialysegerät oder Verfahren nach Anspruch 9, **dadurch gekennzeichnet, dass** das Verbindungsmittel (12) derart ausgebildet ist, dass eine Verbindung des Behälters (10) nur mit einem Konnektionsmittel eines Dialysegerätes möglich ist, wobei vorzugsweise vorgesehen ist, dass das Verbindungsmittel einen ersten Verbinder (120) und einen zweiten Verbinder (122) aufweist, die mit dem Konnektionsmittel konnektierbar sind und die einen oder mehrere Hohlräume, Kanäle, Leitungen oder dergleichen aufweisen, die derart ausgebildet sind, dass durch diese im Betrieb wenigstens eine Flüssigkeit, vorzugsweise Wasser sowie das saure Flüssigkonzentrat oder wenigstens eine Flüssigkeit, vorzugsweise Wasser sowie das saure Flüssigkonzentrat und wenigstens ein Gas, vorzugsweise Luft, strömt.

11. Dialysegerät oder Verfahren nach Anspruch 9 oder 10, **dadurch gekennzeichnet, dass** der Behälter (10) als Standbodenbeutel ausgeführt ist.

12. Dialysegerät oder Verfahren nach einem der Ansprüche 9 bis 11, **dadurch gekennzeichnet, dass** der Behälter (10) wenigstens zwei oder genau zwei Folien aufweist, die die Behälterwandungen bilden, wobei eine der Folien einen im leeren Zustand des Behälters (10) eingefalteten Abschnitt aufweist, der im befüllten Zustand des Behälters (10) eine Behälterwandung bildet.

## Claims

1. A dialysis machine, **characterized in that** the dialysis machine is connected to a container (10) or is suitable for connection to a container (10), wherein the dialysis machine includes means which are formed such that by these means at least one liquid, preferably water, and at least one gas, preferably air, is introduced into the container (10) connected to the dialysis machine, wherein the container (10) contains at least one dry concentrate (20), wherein the dry concentrate (20) is formed such that when dissolved in a liquid, preferably in water, it forms at least one acid liquid concentrate or a part of an acid liquid concentrate which is suitable for preparing at least one dialysis solution, wherein the container (10) is formed such that it includes at least one coupling means (12) by means of which the container (10) is connectable to the dialysis machine, wherein the container (10) includes at least one introduction means (16) by which the liquid and the gas can be introduced into the container (10) for the purpose of dissolving the dry concentrate (20), wherein the introduction means (16) is formed as a hose or comprises at least one hose, wherein it is furthermore provided that the introduction means (16) is dimensioned such that it protrudes down into the dry concentrate (20).

2. The dialysis machine according to claim 1, **characterized in that** the dialysis machine includes means which are formed such that by these means the acid liquid concentrate present in the container (10) connected to the dialysis machine can be or is discharged from the container (10), wherein it is preferably provided that these means partly or completely are the same means by which at least one liquid, preferably water, or the at least one gas, preferably air, can be or is introduced into the container (10) connected to the dialysis machine.

3. The dialysis machine according to claim 1 or 2, **characterized in that** at least one control or regulating unit is provided, which is configured such that it controls or regulates the supply of the liquid and of the gas into the container (10) and/or the discharge of gas, preferably air, and/or of the acid liquid concentrate from the container (10).

4. A dialysis machine, **characterized in that** the dialysis machine includes at least one connecting means (16, 52) by means of which a compressed air guiding means is connected to a liquid guiding means, wherein by means of the connecting means (16, 52) liquid and gas can be supplied to a container (10) containing at least one dry concentrate (20), and wherein by means of the connecting means (16, 52) acid liquid concentrate can be discharged from the container (10).

5. The dialysis machine according to claim 4, **characterized in that** at least one control or regulating unit is provided, which is configured such that it controls or regulates the supply of liquid, preferably water, and of gas, preferably air, into the container (10) and/or the discharge of gas, preferably air, and/or of the acid liquid concentrate from the container (10) and/or that the dialysis machine furthermore has the characterizing features according to any of claims 1 or 2.

6. A method for preparing a liquid concentrate which serves for preparing a dialysis solution, **characterized in that** it is an acid liquid concentrate and that the method comprises the following steps:
a) connecting a container (10) to a dialysis machine,
b) dissolving the dry concentrate (20) in the container (10),
c) discharging the acid liquid concentrate obtained by dissolving the dry concentrate (20) from the container (10), and
d) introducing a liquid, preferably water, and gas, preferably air into the container (10) for the purpose of dissolving the dry concentrate (20),
wherein the container (10) contains at least one dry concentrate (20), wherein the dry concentrate (20) is formed such that when dissolved in a liquid, preferably in water, it forms at least one acid liquid concentrate or a part of an acid liquid concentrate which is suitable for preparing at least one dialysis solution, wherein the container (10) is formed such that it includes at least one coupling means (12) by means of which the container (10) is connectable to the dialysis machine, wherein the container (10) includes at least one introduction means (16) by which the liquid and the gas can be introduced into the container (10) for the purpose of dissolving the dry concentrate (20), wherein the introduction means (16) is formed as a hose or comprises at least one hose, wherein it is furthermore provided that the introduction means (16) is dimensioned such that it protrudes down into the dry concentrate (20)..

7. The method according to claim 6, **characterized in that** gas, preferably air, is discharged from the container (10) and/or that the acid liquid concentrate discharged from the container (10) is used for preparing a dialysis solution.

8. The method according to claim 6 or 7, **characterized in that** the acid liquid concentrate present in the container (10) only is discharged from the container (10) when the dry concentrate (20) present therein is dissolved completely.

9. The dialysis machine according to any of claims 1 to 3 or the method according to any of claims 6 to 8, **characterized in that** in at least one region the container (10) includes wall regions (17, 18) facing towards each other, between which a valley-shaped region (19) or a depression is formed, wherein in the operating position of the container (10) the dry concentrate (20) is at least also present in the valley-shaped region (19) or in the depression.

10. The dialysis machine or method according to claim 9, **characterized in that** the coupling means (12) is formed such that a connection of the container (10) is possible only with a connecting means of a dialysis machine, wherein it is preferably provided that the coupling means includes a first connector (120) and a second connector (122), which are connectable with the connecting means and which include one or more cavities, channels, lines or the like, which are formed such that in operation at least one liquid, preferably water, as well as the acid liquid concentrate or at least one liquid, preferably water as well as the acid liquid concentrate and at least one gas, preferably air, flows through the same.

11. The dialysis machine or method according to claim 9 or 10, **characterized in that** the container (10) is configured as a stand-up pouch.

12. The dialysis machine or method according to any of claims 9 to 11, **characterized in that** the container (10) includes at least two or exactly two films which form the container walls, wherein one of the films has a portion which in the empty condition of the container (10) is folded in and which in the filled condition of the container (10) forms a container wall.

## Revendications

1. Machine de dialyse, **caractérisée en ce que** la machine de dialyse est connectée à un récipient (10) ou convient pour être connectée à un récipient (10), la machine de dialyse comprenant des moyens qui sont conçus de telle sorte que, par l'intermédiaire de ces moyens, au moins un liquide, de préférence de l'eau, et au moins un gaz, de préférence de l'air, est introduit dans le récipient (10) connecté à la machine de dialyse, le récipient (10) contenant au moins un concentré sec (20), le concentré sec (20) étant conçu de telle sorte que lorsqu'il est dissous dans un liquide, de préférence dans l'eau, il forme au moins un concentré liquide acide ou une partie d'un concentré liquide acide, qui convient à la préparation d'au moins une solution de dialyse, le récipient (10) étant conçu de telle sorte qu'il présente au moins un moyen de raccordement (12) au moyen duquel le récipient (10) peut être connecté à la machine de dialyse, le récipient (10) présentant au moins un moyen d'introduction (16), par lequel, dans le but de dissoudre le concentré sec (20), le liquide et le gaz peuvent être introduits dans le récipient (10), le moyen d'introduction (16) étant conçu comme un tuyau ou comprenant au moins un tuyau, et il est en outre prévu que le moyen d'introduction (16) soit dimensionné de telle sorte qu'il fasse saillie dans le concentré sec (20).

2. Machine de dialyse selon la revendication 1, **caractérisée en ce que** la machine de dialyse présente des moyens qui sont conçus de telle sorte que le concentré liquide acide se trouvant dans le récipient (10) connecté à la machine de dialyse peut être ou est évacué du récipient (10) par ces moyens, dans laquelle il est prévu de préférence que ces moyens sont partiellement ou complètement les mêmes moyens par lesquels au moins un liquide, de préférence de l'eau, et au moins un gaz, de préférence de l'air, peuvent être ou sont introduits dans le récipient (10) connecté à la machine de dialyse.

3. Machine de dialyse selon la revendication 1 ou 2, **caractérisé en ce qu'**il est prévu au moins une unité de commande ou de régulation, qui est conçue de telle sorte qu'elle commande ou régule l'alimentation du liquide et du gaz dans le récipient (10) et/ou l'évacuation du gaz, de préférence de l'air et/ou du concentré liquide acide du récipient (10).

4. Machine de dialyse, **caractérisée en ce que** la machine de dialyse présente au moins un moyen de raccordement (16, 52), au moyen duquel un moyen de conduite d'air comprimé est relié à un moyen de conduite de liquide, dans laquelle du liquide et du gaz peuvent être amenés au moyen du moyen de raccordement (16, 52) à un récipient (10) contenant au moins un concentré sec (20) et dans laquelle du concentré liquide acide peut être évacué du récipient (10) au moyen du moyen de raccordement (16, 52).

5. Machine de dialyse selon la revendication 4, **caractérisée en ce qu'**il est prévu au moins une unité de commande ou de régulation, qui est conçue de telle sorte qu'elle commande ou régule l'amenée de liquide, de préférence d'eau, et de gaz, de préférence d'air, dans le récipient (10) et/ou l'évacuation de gaz, de préférence d'air, et/ou du concentré liquide acide du récipient (10) et/ou **en ce que** la machine de dialyse présente en outre les caractéristiques selon l'une quelconque des revendications 1 ou 2.

6. Procédé de préparation d'un concentré liquide utilisé pour préparer une solution de dialyse, **caractérisé en ce qu'**il s'agit d'un concentré liquide acide et que le procédé comprend les étapes suivantes :
a) connecter un récipient (10) à une machine de dialyse,
b) dissoudre le concentré sec (20) dans le récipient (10),
c) évacuer le concentré liquide acide obtenu par dissolution du concentré sec (20) du récipient (10), et
d) introduire un liquide, de préférence de l'eau, et un gaz, de préférence de l'air, dans le récipient (10) afin de dissoudre le concentré sec (20),
dans lequel le récipient (10) contient au moins un concentré sec (20), dans lequel le concentré sec (20) est conçu de telle sorte que, lorsqu'il est dissous dans un liquide, de préférence dans l'eau, il forme au moins un concentré liquide acide ou une partie d'un concentré liquide acide approprié pour produire au moins une solution de dialyse, dans lequel le récipient (10) est conçu de telle sorte qu'il présente au moins un moyen de raccordement (12), au moyen duquel le récipient (10) peut être connecté à la machine de dialyse, le récipient (10) présentant au moins un moyen d'introduction (16) par lequel le liquide et le gaz peuvent être introduits dans le récipient (10) pour dissoudre le concentré sec (20), le moyen d'introduction (16) étant conçu sous forme de tuyau ou comprenant au moins un tuyau, il étant en outre prévu que le moyen d'introduction (16) soit dimensionné de telle sorte qu'il fasse saillie dans le concentré sec (20).

7. Procédé selon la revendication 6, **caractérisé en ce que** du gaz, de préférence de l'air, est évacué du récipient (10) et/ou que le concentré liquide acide évacué du récipient (10) est utilisé pour produire une solution de dialyse.

8. Procédé selon la revendication 6 ou 7, **caractérisé en ce que** le concentré liquide acide contenu dans le récipient (10) n'est évacué du récipient (10) que lorsque le concentré sec (20) contenu dans celui-ci est complètement dissous.

9. Machine de dialyse selon l'une quelconque des revendications 1 à 3 ou procédé selon l'une quelconque des revendications 6 à 8, **caractérisé en ce que** le récipient (10) présente des zones de paroi (17, 18) tournées l'une vers l'autre dans au moins une zone, entre lesquelles est formée une zone en forme de vallée (19) ou une dépression, le concentré sec (20) étant également présent, en position de fonctionnement du récipient (10), au moins dans la zone en forme de vallée (19) ou dans la dépression.

10. Machine ou procédé de dialyse selon la revendication 9, **caractérisé en ce que** le moyen de raccordement (12) est conçu de telle sorte qu'une connexion du récipient (10) n'est possible qu'avec un moyen de connexion d'une machine de dialyse, dans lequel il est prévu de préférence que le moyen de raccordement comprend un premier connecteur (120) et un deuxième connecteur (122), qui peuvent être connectés aux moyens de connexion et qui présentent une ou plusieurs cavités, canaux, conduites ou similaires, qui sont conçus de telle sorte que, pendant le fonctionnement, au moins un liquide, de préférence de l'eau, et le concentré liquide acide ou au moins un liquide, de préférence de l'eau, et le concentré liquide acide et au moins un gaz, de préférence de l'air, les traversent.

11. Machine ou procédé de dialyse selon la revendication 9 ou 10, **caractérisé en ce que** le récipient (10) est conçu comme un sachet à fond plat.

12. Machine ou procédé de dialyse selon l'une quelconque des revendications 9 à 11, **caractérisé en ce que** le récipient (10) présente au moins deux ou exactement deux feuilles qui forment les parois du récipient, l'une des feuilles ayant une section qui est repliée lorsque le récipient (10) est vide et forme une paroi de récipient lorsque le récipient (10) est rempli.
